# EUROPÄISCHE PATENTSCHRIFT

(11) **EP 3 852 841 B1**
(45) Veröffentlichungstag und Bekanntmachung des Hinweises auf die Patenterteilung: **09.12.2026**
(21) Anmeldenummer: 19783703.2
(22) Anmeldetag: 18.09.2019
(51) Int. Cl.: A61M 5/20, A61M 5/24

(54) **ELEKTRONISCHES ZUSATZMODUL FÜR INJEKTIONSGERÄTE**
ELECTRONIC ADD-ON MODULE FOR INJECTORS
MODULE SUPPLÉMENTAIRE ÉLECTRONIQUE POUR APPAREILS D'INJECTION

(30) Priorität: 19.09.2018 EP 18195521
(43) Veröffentlichungstag der Anmeldung: 28.07.2021
(73) Patentinhaber: Ypsomed AG, 3401 Burgdorf (CH)
(72) Erfinder: URBANEK, Leos, 3012 Bern (CH); KALBERMATTER, Gabriel, 3400 Burgdorf (CH)
(74) Vertreter: Meier Obertüfer, Jürg
(86) Internationale Anmeldenummer: PCT/IB2019/057866
(87) Internationale Veröffentlichungsnummer: WO 2020/058875

(56) Entgegenhaltungen:
- WO-A1-2009/152542
- CH-A2- 713 129
- US-A1- 2015 004 582
- US-A1- 2017 357 776
- US-A1- 2018 236 185

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf ein portables elektronisches Zusatzmodul zum Aufsetzen auf ein medizinisches Injektionsgerät.

### HINTERGRUND

Die Patentanmeldung EP 2781230 beschreibt ein auch als Autoinjektor bezeichnetes Injektionsgerät zum automatischen Ausschütten einer medizinischen Substanz mittels einer vorgespannten Injektionsfeder welche über eine Kolbenstange einen Stopfen in eine Spritze drückt. Die Bewegung des Stopfens bewirkt eine Ausschüttung oder Abgabe der Substanz durch eine Nadel an einem distalen Ende der Spritze. Optional kann die Injektionsfeder oder ein weiteres Energiespeicherelement auch eine Einstechbewegung der Spritze relativ zu einem Gehäuse des Injektionsgeräts in distale Richtung automatisch ausführen. Das Injektionsgerät umfasst weiter eine in einer Längsrichtung zwischen einer proximalen und einer distalen Position verschiebbare Nadelschutzhülse. Die Nadelschutzhülse ist mit einer Nadelschutzhülsenfeder als separatem Antriebselement gekoppelt, welche die Nadelschutzhülse nach erfolgter Substanzabgabe in die distale Position schiebt, in welcher sie die Nadel seitlich umgibt oder abschirmt. Ein bewegliches Anschlagelement als Rückkopplungsvorrichtung zur Erzeugung eines akustischen Signals nach Abgabe einer bestimmten Menge an Substanz wird durch die Nadelschutzhülsenfeder zu einem Anschlag hin beschleunigt. Eine zweite Rückkopplungsvorrichtung mit einem durch die Ausschüttfeder beschleunigten Anschlagelement signalisiert den Beginn der Substanzabgabe.

Die Patentanmeldung WO 2018/064784 beschreibt ein portables, wiederverwendbares elektronisches Zusatzmodul zum Aufsetzen auf ein proximales Ende eines Injektionsgerätes. Das Zusatzmodul verfügt über einen induktiven Sensor zur Detektion einer Bewegung einer magnetischen oder elektrisch leitfähigen Komponente des Injektionsgerätes zwischen einer Ausgangs- und einer Zwischenposition, und zwischen der Zwischenposition und einer Endposition während eines Ausschüttvorganges. Dadurch wird eine einfache und zuverlässige Kontrolle und Überwachung eines Injektionsgeräteeinsatzes ermöglicht.

US 2018236185 A1 offenbart ein Zusatzmodul, welches mit einem Injektionsgerät verbindbar ist. Das Zusatzmodul beinhaltet Sensoren für eine Statuserkennung und ein hülsenförmiges Halteelement. Wenn das Injektionsgerät in das Zusatzmodul eingeschoben wird, wird es zwischen Armen des Halteelements und einem beweglichen und vorgespannten Supportelement gehalten.

CH 713129 A2 offenbart ein Zusatzmodul für einen Injektor, welches ein Gehäuse und eine relativ zum Gehäuse bewegliche Aufnahmeeinheit umfasst. Wenn der Injektor in das Zusatzmodul eingeschoben ist, verriegeln Haltearme den Injektor im Gehäuse des Zusatzmoduls. Dabei ist auch die bewegliche Aufnahmeeinheit mittels Sperrnocken gehalten und zum Gehäuse verriegelt.

US 20170357776 A1 beschreibt ein Medikamentensystem, das ein Medikamentengerät und ein Begleitgerät umfasst. Das Begleitgerät umfasst ein internes Teil zur Aufnahme des Medikamentengeräts, welches in Richtung der Längsachse bewegbar ist und ist so konfiguriert, dass es dem Benutzer Informationen über das Medikamentengerät oder dessen Verwendung übermittelt und Komponenten wie Sensoren, einen Mikroprozessor, eine Signalausgabekomponente und eine Stromquelle umfassen kann. Das System bietet dem Benutzer Schulungen, Anleitungen und Feedback zur ordnungsgemäßen Bedienung und Verwendung des Medikamentengeräts.

Durch das Aufsetzen eines Zusatzmoduls auf das Injektionsgerät wird letzterem ein Zusatzgewicht aufgebürdet, und damit die träge Masse derjenigen Gerätekomponente vergrössert, welche das Zusatzmodul trägt. Falls das Injektionsgerät mit aufgesetztem Zusatzmodul vor der Injektion über eine Tischkante zu Boden fallen sollte kann dadurch das Injektionsgerät einer Zusatzbelastung ausgesetzt werden. Ist das Zusatzgerät auf das proximale Ende als Trägerkomponente des Injektionsgerätes aufgesetzt und schlägt ein distales Ende als Aufprallkomponente des Injektionsgerätes auf den Boden auf, werden Übergänge zwischen der Träger- und der Aufprallkomponente ausgeprägt belastet. Falls die Aufprallkomponente gegenüber der Trägerkomponente nur über eine schwach dimensionierte Schnitt- oder Kontaktstelle und somit nicht ausreichend kraftschlüssig verbunden ist, kann eine Beschädigung der Schnittstelle die Folge sein. Dies kann beispielsweise eintreten, wenn das Gehäuse des Injektionsgeräts nur als Trägerkomponente fungiert und der Nadelschutzkappenentferner als Aufprallkomponente gegenüber dem Gehäuse verschiebbar ist und dabei den Spritzenhalter als weitere Gerätekomponente mitbewegt. Der Spritzenhalter kann in der Folge auf einen gehäusefesten Anschlag prallen und dabei Schaden nehmen. Auch kann bei einem Aufprall auf einen Nadelschutzkappenentferner des Injektionsgerätes auch die Nadelschutzhülse gegenüber dem Injektionsgerät beschleunigt und dadurch ein Verriegelungsmechanismus der Nadelschutzhülse beeinträchtigt werden, so dass Letzterer nach dem Injektionsvorgang nicht mehr bestimmungsgemäss funktioniert.

Da das Injektionsgerät durch einen solchen Aufprall nicht zwingend spontan auslöst oder visuell von aussen erkennbare Schäden erleidet wird ein Nutzer das Injektionsgerät unter Umständen noch verwenden wollen ohne sich möglicher Funktionseinschränkungen bewusst zu sein.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere
Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

### DARSTELLUNG DER ERFINDUNG

Es ist eine Aufgabe der Erfindung, eine mechanische Zusatzbelastung einzelner Komponenten eines Injektionsgerätes durch ein auf das Injektionsgerät aufgesetztes Zusatzmodul zu minimieren. Diese Aufgabe wird gelöst durch ein Zusatzmodul und durch eine Verwendung eines Injektionsgeräts mit den Merkmalen der unabhängigen Ansprüche. Bevorzugte Ausführungsformen der Erfindung sind Gegenstand der abhängigen Ansprüche.

Erfindungsgemäss wird ein elektronisches Zusatzmodul vor Injektionsbeginn auf ein Injektionsgerät mit einer Längs- oder Hauptachse, welche ein einstechseitiges distales Ende mit einem gegenüberliegenden proximalen Ende des Injektionsgeräts verbindet, lösbar aufgesetzt oder aufgesteckt. Das Zusatzmodul umfasst ein Sensorelement zur Detektion eines Zustandes oder Vorganges im Injektionsgerät, ein Prozessorelement zur Auswertung und/oder Verarbeitung eines Signals des Sensorelements, einen Energiespeicher zur Energieversorgung des Sensor- und/oder des Prozessorelementes, und optional eine Kommunikationseinheit zur drahtlosen Kommunikation von Daten des Prozessorelements. Das Zusatzmodul weist ein erstes Modulteil auf, welches in Richtung der Längsachse durch einen Haltemechanismus im Rahmen von Fertigungstoleranzen axialfest mit dem Injektionsgerät verbindbar ist und im aufgesetzten Zustand axialfest mit dem Injektionsgerät verbunden ist. Das Zusatzmodul weist zudem ein zweites Modulteil auf, welches zumindest in einer distalen Richtung parallel zur Längsachse für eine Relativbewegung gegenüber dem ersten Modulteil, für eine gedämpfte Verzögerungs- oder Abbremsbewegung, beweglich ist. Erfindungsgemäss bleibt das erste Modulteil während der Relativbewegung axialfest mit dem Injektionsgerät verbunden und der Haltemechanismus bleibt unabhängig von der Relativbewegung bis zur Betätigung einer Löseeinheit wirksam, die Relativbewegung führt also nicht etwa zur Lösung des Zusatzmoduls vom Injektionsgerät. Der Haltemechanismus zur axialfesten Verbindung von Injektionsgerät und erstem Modulteil ist
formschlüssig und umfasst dazu beispielsweise Halteelemente am ersten Modulteil, welche formschlüssig in Aussparungen im Injektionsgerät eingreifen, so dass der Haltemechanismus insbesondere keine proximal des ersten Modulteils und koaxial zur Längsachse positionierte Wendelfeder zum kraftschlüssigen Anpressen des ersten Modulteils an das Injektionsgerät aufweist.

Durch die erfindungsgemässe Relativbewegung zwischen zwei Modulteilen wird die Kraft- und/oder Energieübertragung zwischen Zusatzmodul und Injektionsgerät in Richtung der Längsachse kontrolliert und insbesondere ein Maximalkraftstoss auf das Injektionsgerät verzögert und bevorzugt auch begrenzt. Das zweite Modulteil kann am Ende einer Relativbewegung mit einer Amplitude zwischen 0.2 und 10 Millimetern, und bevorzugt zwischen 0.5 und 5 Millimetern, auf einen Endanschlag am ersten Modulteil oder am Injektionsgerät aufprallen, und/oder über gegenüber der Längsachse schräggestellte Flächen mit dem ersten Modulteil oder dem Injektionsgerät verklemmen. Somit tritt eine maximale Wirkung der trägen Masse des zweiten Modulteils erst am Ende der Relativbewegung und damit gegenüber der unmittelbaren Wirkung der trägen Masse des ersten Modulteils verzögert auf. Die Belastung der Trägerkomponente des Injektionsgerätes, welche mit dem ersten Modulteil axialfest verbunden ist, und der Schnittstellen zwischen der Trägerkomponente und weiteren Komponenten des Injektionsgerätes wird dadurch mit geringerer Intensität über die Relativbewegung zeitlich verteilt.

Gemäss der Erfindung weist das Zusatzmodul ein von den Modulteilen verschiedenes Dämpfungselement auf zur Dämpfung der Relativbewegung zwischen den Modulteilen. Das Dämpfungselement kann unmittelbar zwischen den beiden Modulteilen vorgesehen sein, oder zwischen dem zweiten Modulteil und dem Injektionsgerät wirken. Das Dämpfungselement kann so ausgelegt sein, dass es die kinetische Energie des zweiten Modulteils vollständig absorbieren kann. Alternativ kann das Dämpfungselement gesättigt werden, beispielsweise durch eine maximale Kompression einer Druckfeder, wobei anschliessend wie im Falle eines expliziten Endanschlags die Verzögerungskraft des zweiten Modulteils durch die Trägerkomponente des Injektionsgeräts übernommen wird. Bevorzugt umfasst das Dämpfungselement eine elastische Federkomponente zur Speicherung von potentieller Energie und/oder eine unelastische Dissipationskomponente zur Vernichtung der kinetischen Energie des zweiten Modulteils und zur bevorzugt aperiodischen Schwingungsdämpfung. Die Dissipation erfolgt beispielsweise durch einen Reibschluss der Federkomponente mit einer Wandung oder durch ein integriertes oder separates Reibungselement. Eine vollständig unelastische Dämpfung mit einer plastischen
Verformung des Dämpfungselements kann zumindest für eine einmalige Schadenprävention am Injektionsgerät ebenfalls dienlich sein.

Für die Zwecke der vorliegenden Erfindung umfasst der Begriff der Dämpfung auch eine näherungsweise elastische Federung, so dass kinetische Energie des zweiten Modulteils während der Verzögerung durch die Dämpfung vernichtet und/oder durch die Federung in potentielle Energie umgewandelt wird. Das zweite Modulteil kann am Ende eines gedämpften Verzögerungshubs mit einer Amplitude zwischen ein und zehn, und bevorzugt zwischen zwei und vier, Millimetern auf einen Verzögerungsendanschlag am ersten Modulteil oder am Injektionsgerät aufprallen, und/oder über gegenüber der Längsachse schräggestellte Flächen mit dem ersten Modulteil oder dem Injektionsgerät verklemmen. Somit wird die Verzögerungskraft der trägen Masse des zweiten Modulteils zeitlich über eine Dauer der Verzögerungsbewegung verteilt und entsprechend abgedämpft oder moderiert übertragen. Die Belastung der Trägerkomponente des Injektionsgerätes, welche mit dem ersten Modulteil axialfest verbunden ist, und der Schnittstellen zwischen der Trägerkomponente und weiteren Komponenten des Injektionsgerätes wird dadurch gemildert.

In einer bevorzugten Variante der Erfindung ist die träge Masse des zweiten Modulteils grösser als die träge Masse des ersten Modulteils. Insbesondere ist der Energiespeicher eine Komponente des zweiten Modulteils.

In einer vorteilhaften Ausführungsform der Erfindung ist das Sensorelement im ersten Modulteil angeordnet. Dadurch wird sichergestellt, dass das Sensorelement axial bestimmungsgemäss platziert und auf die zu detektierende Signalkomponente des Injektionsgerätes ausgerichtet ist. Ein axialer Abstand in Längsrichtung zwischen dem Sensorelement und der Signalkomponente hängt somit während dem Ausschüttvorgang nicht etwa von einer Haltekraft des Nutzers ab, welcher das Zusatzmodul am zweiten Modulteil greift und auf die Injektionsstelle drückt. Zudem werden Vibrationen von Komponenten des Injektionsgerätes auf einen Beschleunigungssensor oder ein Gyroskop als Sensorelement im ersten, mit dem Injektionsgerät fest verbundenen Modulteil besser übertragen. Das Sensorelement produziert bevorzugt ein analoges elektrisches Sensorsignal, welches über eine flexible Verbindung unabhängig von besagtem axialem Abstand an eine Sensorkontrolleinheit der Prozessorelemente übertragen wird. Die Sensorelemente sorgen für eine berührungsfreie induktive, kapazitive, optische, akustische, oder auch für eine kontaktbasierte Detektion einer Position oder eines Zustandes von Komponenten des Injektionsgeräts. Alternativ können axialverschiebungsverträgliche Sensorelemente auch am zweiten Modulteil, insbesondere in einem Gehäuseeinsatz oder in der Elektronikeinheit, montiert und über starre Leiterbahnen mit der Prozessoreinheit verbunden werden.

In einer bevorzugten Ausführungsform der Erfindung weist das zweite Modulteil einen Anschlag oder eine Anschlagsfläche auf, welcher am Ende einer durch ein oder das Dämpfungselement verzögerten Verzögerungs- oder Abbremsbewegung auf einen Verzögerungsendanschlag oder eine Gegenanschlagsfläche des Injektionsgerätes oder des ersten Modulteils aufschlagen oder aufprallen kann. Durch diesen Aufschlag wird die Amplitude oder der Hub der Verzögerungsbewegung begrenzt. Entsprechend können das Dämpfungselement eine geringere Dämpfungskonstante und/oder der Verzögerungshub eine geringere Länge aufweisen als in einer Ausführungsform mit einem für die vollständige Absorption der kinetischen Energie des zweiten Modulteils ausgelegten Dämpfungselement. Dieser Anschlag kann gleichzeitig auch eine Haltekraft des Nutzers aufnehmen, welcher das Zusatzmodul am zweiten Modulteil greift und auf die Injektionsstelle drückt.

Das Dämpfungselement ist bevorzugt eine Helix- oder Wendelfeder, welche beim Aufsetzen des Zusatzmoduls auf das Injektionsgerät vorgespannt wird und somit auch als Aufnahme- oder Aufsetzfeder dient. Dabei werden die beiden Modulteile um einen Aufnahmeweg gegeneinander verschoben, wobei das Dämpfungselement in axialer Richtung komprimiert oder gedehnt wird. Das vorgespannte Dämpfungselement wird durch die Fixierung des zweiten Modulteils in der vorgespannten Position gegen eine Entspannung verrastet, ist aber für eine erfindungsgemässe Dämpfung in Längsrichtung weiter spannbar. Als Dämpfungselement ebenfalls denkbar wären andere Federn wie beispielsweise eine aus einem Federdraht geformte Bügelfeder, eine Blattfeder, ein Federelement aus Kunststoff, oder auch ein unelastisches Dämpfungselement.

In einer Weiterbildung der Erfindung umfasst das erste Modulteil einen Innenraum für eine gekapselte Aufnahme des zweiten Modulteils, und das zweite Modulteil, bevorzugt zumindest der Energiespeicher, führt die Verzögerungsbewegung innerhalb dieses Innenraums aus. Das erste Modulteil weist also eine an die Dimensionen des zweiten Modulteils angepasste Ausdehnung in radialer Richtung auf. Das erste Modulteil definiert ein Aufnahmevolumen für das Injektionsgerät und kann ein Halteelement mit elastischen Modulflügeln zum seitlichen Aufschnappen oder axialen Aufschieben des Zusatzmoduls auf das Injektionsgerät umfassen.

In alternativen Weiterbildungen der Erfindung umfasst das erste Modulteil eine innenliegende Hülse als Aufnahmeeinheit für das Injektionsgerät, welche das aufgenommene Injektionsgerät in axialer Richtung auf einer Länge von mindestens zwei cm und bevorzugt mindestens fünf cm allseits umgibt, und das zweite Modulteil ein aussenliegendes Modulgehäuse welches die innenliegende Hülse zumindest teilweise umgibt. Dabei weist das zweite Modulteil bevorzugt einen Griff oder eine Griffposition zur Ergreifung von Zusatzmodul und Injektionsgerät auf mit einer Ausdehnung in Richtung der Längsachse entsprechend mindestens einer halben Handbreite eines Benutzers. Diese Griffposition überdeckt und ersetzt vorzugsweise eine Griffposition des Injektionsgerätes, welche bei einer Verwendung des Injektionsgerätes ohne Zusatzmodul vom Nutzer gewählt würde. Alternativ kann das zweite Modulteil gegenüber dem ersten Modulteil vornehmlich axial versetzt sein, und der Griff am ersten Modulteil vorgesehen sein. Der Haltemechanismus umfasst bevorzugt seitlich an der innenliegenden Hülse angebrachte Halteelemente zum radialen Eingreifen in seitliche Aussparungen des Injektionsgerätes. Entsprechend hat die innenliegende Hülse in Längsrichtung eine Ausdehnung, welche mindestens der Distanz zwischen dem proximalen Ende des Injektionsgerätes und der Position der Aussparungen entspricht, wobei bevorzugt besagte Aussparungen von allfälligen Sichtfenstern im Injektionsgerät verschieden und proximal davon vorgesehen sind.

Weiter bevorzugt ist das Zusatzmodul im gekoppelten Zustand so platziert dass eine nach distal gerichtete Anschlagsfläche oder Anschlagskante des zweiten Modulteils einer nach proximal gerichteten Verzögerungsendanschlagsfläche oder -kante eines Nadelschutzkappenentferners gegenüberliegt und an diese am Ende der gedämpften Relativbewegung der Modulteile anschlägt. Dadurch wird die verbleibende kinetische Energie des zweiten Modulteils des Zusatzmoduls über den Nadelschutzkappenentferner abgeleitet und die Trägerkomponente des Injektionsgeräts entlastet. Der Verzögerungsweg oder die Amplitude des Verzögerungshubs und die Dämpfung desselben können tatsächlich bis gegen Null oder zumindest bis auf Fertigungstoleranzen oder bis auf ein für radiale Bewegungen eines Halteelements notwendiges Minimum reduziert werden. Alternativ, und insbesondere bei axial weniger ausgedehnten Zusatzmodulen, fungiert eine proximal orientierte Fläche oder Kante des Injektionsgeräts oder des ersten Modulteils als Verzögerungsendanschlag für das zweite Modulteil.

In weiter bevorzugten Ausbildungen der Erfindung umfasst das erste Modulteil ein Halteelement, insbesondere einen Haltearm mit einem Vorsprung zum Eingreifen in eine Aussparung des Injektionsgerätes, und das zweite Modulteil eine Löseeinheit als zur Begrenzung einer Bewegung der Aufnahmeeinheit in distaler Richtung gegenüber dem Modulgehäuse. Die Löseeinheit umfasst insbesondere einen Löseknopf mit einer in radialer Richtung, senkrecht zur Längsachse beweglichen Betätigungsfläche. In einer Haltekonfiguration verhindert die Löseeinheit eine Freigabebewegung der Aufnahmeeinheit, und das Halteelement hält das Injektionsgerät axialfest in der Aufnahmeeinheit. Bevorzugt ist das Halteelement durch das Modulgehäuse in der Haltekonfiguration verriegelt. Das Zusatzmodul gibt in einer Freigabekonfiguration die Aufnahmeeinheit von der Löseeinheit und das Injektionsgerät vom Halteelement frei. Das Zusatzmodul ist durch eine Freigabebewegung der Aufnahmeeinheit von der Haltekonfiguration in die Freigabekonfiguration und durch eine Aufnahmebewegung von der Freigabekonfiguration zurück in die Haltekonfiguration verstellbar.

Weiterhin umfasst die Löseeinheit einen Führungsnocken und die Aufnahmeeinheit einen Sperrnocken, wobei in der Haltekonfiguration der Führungsnocken derart mit dem Sperrnocken zusammenwirkt, dass die Aufnahmeeinheit an einer Bewegung in distaler Richtung gegenüber dem Gehäuse gehindert ist. Die Aufnahmeeinheit bleibt aber für eine Verzögerungsbewegung unter weitergehender Komprimierung der Aufnahmefeder beweglich. Der Sperrnocken kann auch am Injektionsgerät vorgesehen sein, an einer Stelle welche mit dem Führungsnocken in Kontakt treten kann, bevorzugt durch eine Ausnehmung in der Aufnahmeeinheit hindurch. Im Weiteren ist der Führungsnocken entlang eines Sicherungswegs verschiebbar und von einer Haltestellung, in welcher der Führungsnocken mit dem Sperrnocken zusammenwirkt, in eine Freigabestellung, in der der Sperrnocken vom Führungsnocken freigegeben ist, verstellbar. Der Führungsnocken ist durch eine Löseknopffeder als elastisches Sicherungselement in seine Haltestellung vorspannbar und in dieser gesichert.

In einer weiteren Ausführungsform der Erfindung umfasst das Zusatzmodul eine Freigabeunterstützung für die Freigabebewegung in Form eines elastischen Federelements, welches beim Betätigen einer oder der Löseeinheit zum Entfernen des Injektionsgerätes zuerst gespannt oder geladen wird, und sich anschliessend als Folge derselben Betätigungsbewegung entspannt. Dadurch wird die zuvor aufgenommene Energie wieder abgegeben für die Freigabebewegung der Aufnahmeeinheit in distaler Richtung zur Freigabekonfiguration. Durch diese unterstützte Freigabebewegung werden die Halteelemente aus ihrem Eingriff in das Injektionsgerät gelöst, so dass das Injektionsgerät anschliessend ohne weiteren Kraftaufwand durch den Benutzer vom Zusatzmodul entfernt werden kann. Ebenso kann durch diese Bewegung eine Anzeigekante zur Signalisierung der Freigabekonfiguration sichtbar werden.

Eine Freigabeunterstützung wie vorgehend beschrieben zur finalen Entfernung oder Entnahme des Injektionsgeräts aus dem Zusatzmodul kann auch unabhängig von der beanspruchten Relativbewegung im aufgenommenen oder eingesetzten Zustand des Injektionsgeräts hilfreich sein. Insbesondere wenn das Injektionsgerät zumindest annähernd vollständig von einem Modulgehäuse des Zusatzmoduls umgeben ist und/oder wenig geeignete Griffpositionen aufweist, über welche ein Benutzer Kräfte zum Lösen von Halteelementen aus ihrem Eingriff mit dem Injektionsgerät aufwenden könnte. Oder falls ein Dämpfungselement zur Dämpfung der Relativbewegung vorgesehen ist, aber bei Einsetzen des Injektionsgeräts nicht gespannt wird oder aus anderen Gründen als Freigabeunterstützung nicht zur Verfügung steht.

Ein dergestalt erweitertes elektronisches Zusatzmodul wird vor Injektionsbeginn auf ein Injektionsgerät mit einer Längs- oder Hauptachse, welche ein einstechseitiges distales Ende mit einem gegenüberliegenden proximalen Ende des Injektionsgeräts verbindet, lösbar aufgesetzt oder aufgesteckt. Das Zusatzmodul umfasst ein Sensorelement zur Detektion eines Zustandes oder Vorganges im Injektionsgerät, ein Prozessorelement zur Auswertung und/oder Verarbeitung eines Signals des Sensorelements, einen Energiespeicher zur Energieversorgung des Sensor- und/oder des Prozessorelementes, und optional eine Kommunikationseinheit zur drahtlosen Kommunikation von Daten des Prozessorelements. Das Zusatzmodul weist ein Modulgehäuse auf, welches in Richtung der Längsachse durch einen Haltemechanismus im Rahmen von Fertigungstoleranzen axialfest mit dem Injektionsgerät verbindbar ist und im aufgesetzten Zustand axialfest mit dem Injektionsgerät verbunden ist. Das Zusatzmodul weist eine Löseeinheit auf zum Lösen des Haltemechanismus und zur Bewegung des Injektionsgeräts in eine Freigaberichtung. Erfindungsgemäss ist eine Freigabeunterstützung für die Freigabebewegung in Form eines elastischen Federelements vorgesehen, welches beim Betätigen der Löseeinheit zum Entfernen des Injektionsgerätes zuerst gespannt oder geladen wird, und sich anschliessend als Folge derselben Betätigungsbewegung entspannt. Dadurch wird die zuvor aufgenommene Energie wieder abgegeben für die Freigabebewegung Injektionsgeräts in distaler Richtung.

Ein Zusatzmodul nach der Erfindung kann auch aufweisen eine Kommunikationseinheit zur drahtlosen Kommunikation mit einem mobilen Gerät, beispielsweise einem Mobiltelefon oder Smartphone, und/oder einen optischen, akustischen, oder taktilen Zustandsanzeiger. Ein angezeigter Zustand kann einen Gerätezustand des Injektionsgerätes, einen Modulzustand des Zusatzmoduls, oder einen Vorgangszustand eines laufenden oder abgeschlossenen Injektionsvorganges umfassen. Der Zustandsanzeiger des Zusatzmoduls kann einfach gehalten werden und sich auf einige wenige LEDs beispielsweise in Ampelfarben und/oder einen akustischen Signalgenerator zur Erzeugung von sprachunabhängigen Geräuschen oder Melodien beschränken. Dies ist insbesondere im Zusammenwirken mit den fortgeschrittenen graphischen Anzeigemöglichkeiten und Sprachausgabemöglichkeiten eines Smartphones vorteilhaft, da das drahtlos an das Zusatzmodul gekoppelte Smartphone die verfeinerte, über eine Statusanzeige hinausgehende Kommunikation mit dem Benutzer übernimmt. Die Zustandsinformation kann eine Angabe zum Ablauf einer Haltezeit oder Wartezeit umfassen, welche der Benutzer nach abgeschlossener Ausschüttung abwarten muss bevor das Injektionsgerät von der Injektionsstelle sicher entfernt werden kann. Eine einfache Erfassung der seit dem festgestellten Ausschüttende verflossenen Zeit und ein Vergleich mit einer Solldauer erlaubt es dem Benutzer den Zeitpunkt zum sicheren Entfernen des Injektionsgerätes anzeigen. Dies kann sowohl durch ein Zusatzmodul mit Zeiterfassungsfunktion als auch durch ein mit Echtzeit-Ereignisübertragung an das Zusatzmodul gekoppeltes mobiles Gerät erfolgen.

Ein Zusatzmodul nach der Erfindung kann in vorteilhafter Weise wiederholt eingesetzt werden zur Überwachung eines Gebrauchs oder Einsatzes von automatischen Einweg-Injektionsgeräten oder Autoinjektoren. Insbesondere ist das Zusatzmodul geeignet für einen Retrofit-Einsatz bei existierenden Injektionsgeräten, welche für eine Anpassung oder Modifikation nicht zur Verfügung stehen. In dieser Konfiguration sind im Injektionsgerät keine Sensoren vorgesehen zur Erfassung oder Bearbeitung von Sensordaten über die Operation des Gerätes, ebenso wenig eine Kommunikationsschnittstelle zur Übermittlung dieser Daten an einen Empfänger. Dementsprechend müssen die Sensorelemente im Zusatzgerät derart ausgestaltet und positioniert werden, dass sie Zustandsänderungen oder Primärsignale von innerhalb des Injektionsgerätes feststellen können.

Für den Fachmann sind direkt und offensichtlich weitere Ausführungsformen und Ausgestaltungen erkennbar, welche sich aus Kombinationen der beschriebenen Beispiele oder Kombinationen der beschriebenen Beispiele mit dem allgemeinen Fachwissen des Fachmanns ergeben.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
Fig.1 zeigt ein Zusatzmodul und ein Injektionsgerät in perspektivischer Ansicht;
Fig.2 zeigt schematisch drei Varianten der Erfindung im Ausgangszustand;
Fig.3 skizziert einen Krafteinwirkungsverlauf im Falle eines Aufpralls des Injektionsgeräts;
Fig.4 zeigt die Einzelteile des Zusatzmoduls in perspektivischer Ansicht;
Fig.5 vergrössert Einzelteile aus Fig.4 mit weiteren Details;
Fig.6 zeigt einen ersten Aus-Schnitt durch Zusatzmodul und Injektionsgerät,
Fig.7 zeigt einen zweiten Aus-Schnitt durch das Zusatzmodul,
Fig.8 zeigt eine Schnittansicht des Zusatzmoduls in einer Freigabekonfiguration;
Fig.9 zeigt einen mittleren Bereich des Zusatzmoduls in der Freigabekonfiguration;
Fig.10 zeigt eine Schnittansicht des Zusatzmoduls in einer Haltekonfiguration;
Fig.11 zeigt einen mittleren Bereich des Zusatzmoduls in der Haltekonfiguration
Fig.12 zeigt eine erste Variante einer spannbaren Auswurfshilfe, und
Fig.13 zeigt eine zweite Variante einer spannbaren Auswurfshilfe.

### FIGURENBESCHREIBUNG

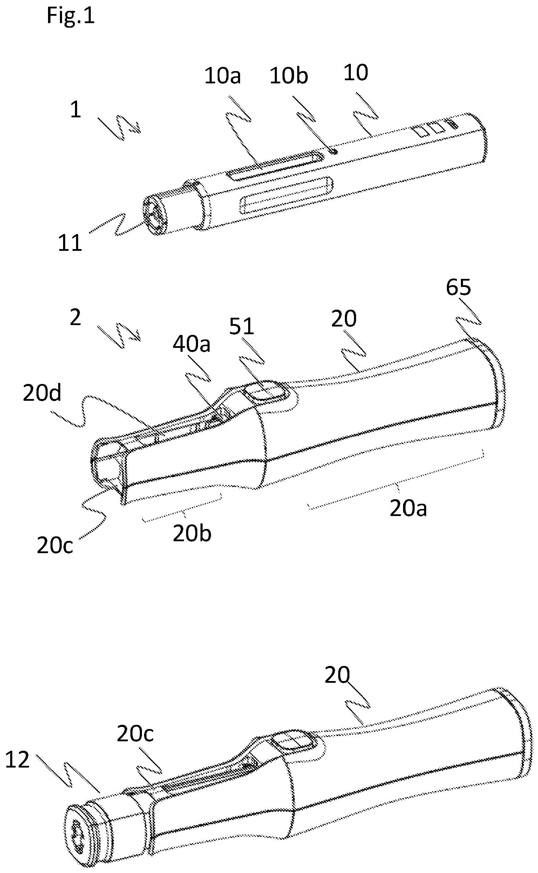

Fig.1 zeigt ein Injektionssystem mit einem Injektionsgerät 1 und einem Zusatzmodul 2, sowohl in getrenntem (Fig.1 oben) als auch in aufgesetztem oder gekoppeltem (Fig.1 unten) Zustand. Das Injektionsgerät weist ein längliches, um eine Längsachse symmetrisches Gerätegehäuse 10 auf mit Fenstern 10a welche die Sicht auf einen Spritzenkörper freigeben, Aussparungen 10b welche für eine Arretierung des Zusatzmoduls genutzt werden, und einer Nadelschutzhülse 11 welche zwischen einer ersten, eine Nadel des Injektionsgerätes abschirmenden Position (in Fig.1 gezeigt) und einer zweiten, die Nadel freigebenden Position verschiebbar ist. Das eingesetzte Injektionsgerät ist mit einem Nadelschutzkappenentferner 12 dargestellt, welcher im Auslieferungszustand auf ein distales Ende des Injektionsgerätes montiert ist und vor der Injektion zusammen mit einer die Nadel mechanisch schützenden Nadelschutzkappe entfernt oder abgezogen werden muss.

Das Zusatzmodul hat ein längliches, hülsenförmiges Modulgehäuse 20 mit einem Innenraum als Geräteaufnahme für das Injektionsgerät, welcher an eine äussere Form des Gerätegehäuses 10 als Trägerkomponente des Injektionsgerätes abgestimmt ist, so dass zur Kopplung das Injektionsgerät in das Zusatzmodul eingeschoben werden kann. Das Zusatzmodul weist eine Betätigungsfläche 51 zum Entkoppeln von Injektionsgerät und Zusatzmodul und an seinem proximalen Ende einen Lichtleiter 65 als optische Zustandsanzeige für eine visuelle Rückmeldung an den Benutzer auf. Das Modulgehäuse 20 umfasst eine leicht konkave Griffposition 20a, welche distal durch eine leichte Erhöhung oder einen Bereich mit vergrössertem Durchmesser zur erleichterten Aufnahme einer Benutzerkraft in distale Richtung begrenzt ist.

In distaler Richtung weist das Zusatzmodul zwei Laschen oder Längsstege 20b auf, welche an ihrem distalen Ende leicht nach aussen gewölbt und untereinander durch stabilisierende Querstege 20c verbunden sind. Zwischen den Laschen und begrenzt durch die Querstege sind seitliche Längsöffnungen 20d gebildet, welche im gekoppelten Zustand mit den Fenstern 10a im Gerätegehäuse ausgerichtet sind und den Blick auf eine im Injektionsgerät gelagerte Substanz ermöglichen. In einem Zustand ohne eingesetztes Injektionsgerät ist eine Anzeigekante 40a einer längsverschiebbaren Aufnahmeeinheit in einer der Längsöffnungen 20d erkennbar. Durch das eingesetzte Injektionsgerät wird diese Anzeigekante nach proximal in das Modulgehäuse verschoben. Eine distale Endfläche der Laschen 20b liegt einer proximalen Kante des Nadelschutzkappenentferners 12 in einem Abstand von wenigen Millimetern in axialer Richtung gegenüber.

Das Gerätegehäuse 10 und das Modulgehäuse 20 weisen im Querschnitt näherungsweise eine quadratische Form auf, wobei jede Seite des Quadrats leicht nach aussen gewölbt ist. In der nachfolgenden Beschreibung wird der Einfachheit halber eine Richtung von der mittigen Längsachse der Gehäuse nach aussen als radiale Richtung bezeichnet.

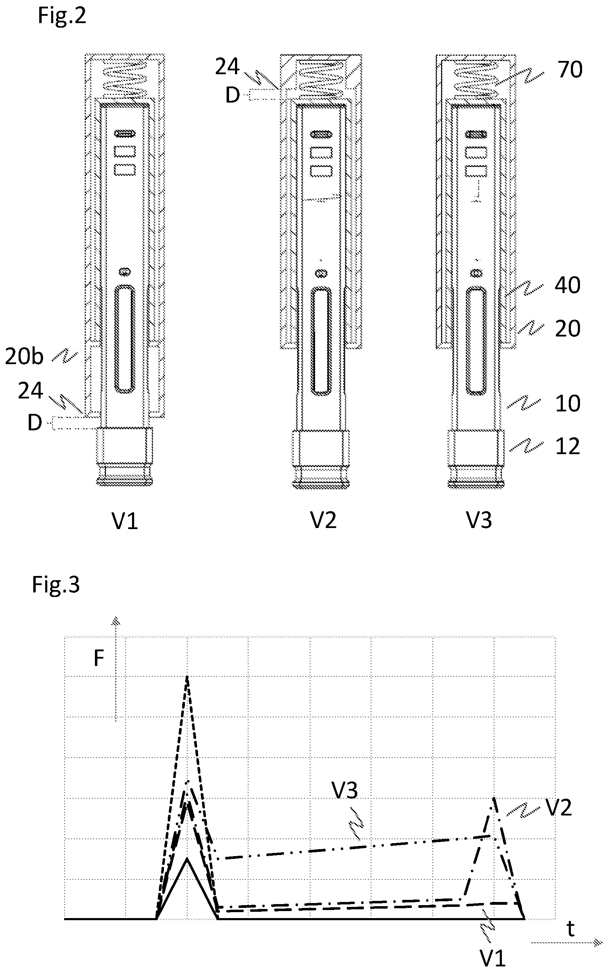

Fig.2 zeigt drei unterschiedliche Varianten V1, V2, V3 der Erfindung jeweils im Ausgangszustand vor einem Abzug der Nadelschutzkappe. Für jede Variante ist dargestellt jeweils eine Aufsicht eines Injektionsgerät mit Gerätegehäuse 10 und Nadelschutzkappenentferner 12 sowie ein Schnitt durch ein aufgesetztes Zusatzmodul mit einem ersten Modulteil als Aufnahmeeinheit 40, axialfest verbunden mit dem Gerätegehäuse 10 als Trägerkomponente, und mit einem zweiten Modulteil umfassend ein Modulgehäuse 20. Die beiden Modulteile sind über ein Dämpfungselement 70 verbunden beziehungsweise elastisch gekoppelt.

Fig.3 zeigt schematisch einen zeitlichen Verlauf der Krafteinwirkung auf die Trägerkomponente, beginnend mit einer Initialbelastung beim Aufprall des Nadelschutzkappenentferners 12, und gefolgt von einer Verzögerungsbelastung während einer Relativbewegung der beiden Modulteile, und von einer Finalbelastung. Der Kraftverlauf ist dargestellt für das Injektionsgerät ohne aufgesetztes Zusatzmodul (minimale Initialbelastung), für ein aufgesetztes einteiliges Zusatzmodul (maximale Initialbelastung), sowie für jede der drei erfindungsgemässen Varianten (reduzierte Initialbelastung gefolgt von Verzögerungsbelastung).

Variante eins (V1, Fig.2 links) entspricht der in Fig.1 gezeigten Konfiguration, bei welcher das zweite Modulteil über ausgedehnte Längsstege 20b verfügt und so angeordnet ist, dass eine nach distal gerichtete Anschlagsfläche 24 des Längsstegs einer nach proximal gerichteten Verzögerungsendanschlagsfläche des Nadelschutzkappenentferners in einem Abstand D von wenigen Millimetern gegenüberliegt. Nach einem gedämpften Verzögerungshub mit Amplitude D treffen die Anschlagsflächen aufeinander, der Verzögerungshub oder die Bremsbewegung des zweiten Modulteils wird also durch einen Endanschlag des Nadelschutzkappenentferners begrenzt. Bei Aufliegen der zwei Anschlagsflächen tritt keine zusätzliche Belastung der Trägerkomponente auf, die Finalbelastung verbleibt also wie in Fig.3 dargestellt auf dem Niveau der Verzögerungsbelastung.

In Variante zwei (V2, Fig.2 Mitte) ist das Zusatzmodul kürzer als in Variante eins und nicht mit ausgedehnten distalen Längsstegen versehen, oder der Nadelschutzkappenentferner aus Variante eins ist vorgängig entfernt worden. Eine nach distal gerichtete Anschlagsfläche 24 im Innern des Modulgehäuses liegt einer nach proximal gerichteten Verzögerungsendanschlagsfläche des ersten Modulteils in einem Abstand D von wenigen Millimetern gegenüber. Die gedämpfte Relativbewegung der Modulteile endet also nach einem Verzögerungsweg D, wonach die Verzögerungskraft des zweiten Modulteils durch die Trägerkomponente übernommen wird. Dies führt zu einer in Fig.3 dargestellten gegenüber dem Niveau der Verzögerungsbelastung erhöhten Krafteinwirkung auf die Trägerkomponente. In Variante zwei dient der Anschlag 24 auch zur Aufnahme einer Haltekraft des Nutzers, welcher das Zusatzmodul am zweiten Modulteil greift und auf die Injektionsstelle drückt.

In Variante drei (V3, Fig.2 rechts) ist ein elastisches Dämpfungselement mit einer erhöhten Dämpfung oder Federkonstanten und/oder einem langen Verzögerungs- oder Federweg zwischen den zwei Modulteilen vorgesehen. Dadurch wird die kinetische Energie des zweiten Modulteils vollständig im elastischen Dämpfungselement absorbiert, und es findet kein harter Anschlag zwischen den zwei Modulteilen mehr statt. Die Kraftübertragung zwischen den zwei Modulteilen wird besser über die Zeit verteilt, und die Verzögerungsbelastung auf die Trägerkomponente bleibt bis zur maximalen Kompression des Federelementes auf einem gegenüber den Varianten eins und zwei erhöhten Niveau, wie in Fig.3 dargestellt.

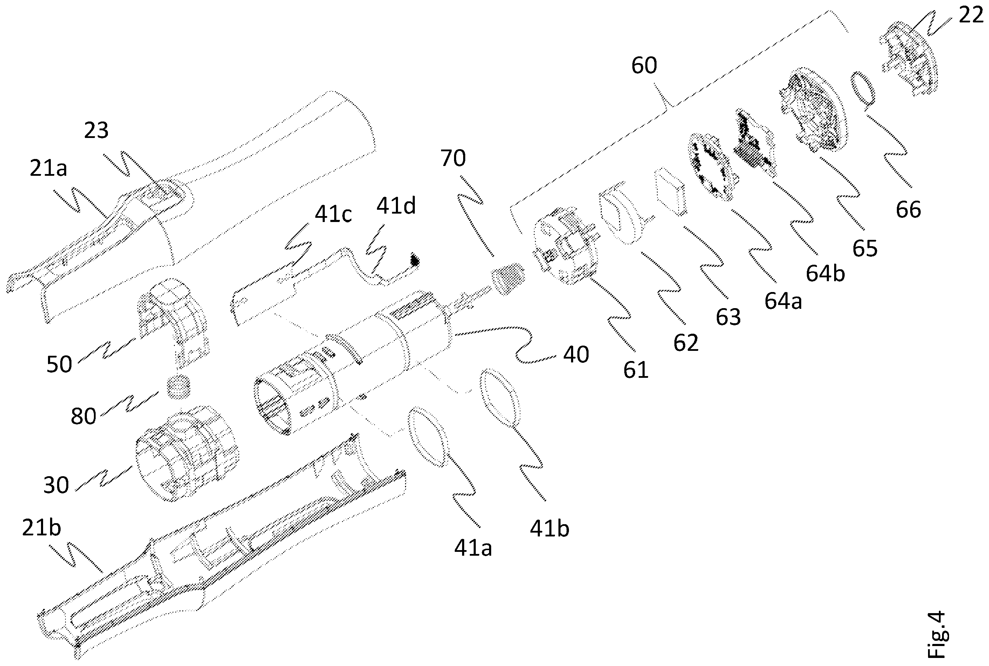

Fig.4 stellt die Einzelteile eines erfindungsgemässen Zusatzmoduls 2 gemäss einer der Varianten eins bis drei in perspektivischer Ansicht dar. Das Zusatzmodul umfasst ein mehrteilig ausgebildetes Gehäuse mit einer oberen Gehäusehälfte 21a, einer damit verbundenen unteren Gehäusehälfte 21b und einem Gehäuseabschluss 22, weiter einen Gehäuseeinsatz 30, eine Aufnahmeeinheit 40, einen Löseknopf 50, eine Elektronikeinheit 60, ein Dämpfungselement 70 und eine Löseknopffeder 80.

Die obere und die untere Gehäusehälfte 21a, 21b weisen je eine annähernd halbrunde Form auf und sind unlösbar verschnappt und/oder mittels Ultraschallschweissen, Laserschweissen, Kleben oder Verstiften miteinander verbunden. Durch die Form der Gehäusehälften entsteht zwischen ihnen ein Innenraum. An einem proximalen Ende wird der Innenraum durch den Gehäuseabschluss 22 oder eine Abschlusswandung zwischen den Gehäusehälften begrenzt. Am distalen Ende bilden die Gehäusehälften eine Öffnung zum Innenraum. Im Innenraum befinden sich axial unbeweglich der Gehäuseeinsatz 30, der Löseknopf 50 und die Elektronikeinheit 60. Die obere Gehäusehälfte 21a weist in einem mittleren Bereich zwischen der Griffposition 20a und dem Steg 20b eine Radialöffnung 23 auf, durch die im montierten Zustand eine Betätigungsfläche 51 des Löseknopfs 50 hindurch ragt.

Die Aufnahmeeinheit 40 ist im Gehäuse wie nachfolgend beschrieben axial beweglich gelagert, und umfasst weiter Sensorelemente in Form von zwei induktiven Sensorspulen 41a, 41b, welche in entsprechenden umlaufenden Nuten in einer Aussenfläche der Aufnahmeeinheit angeordnet sind. Die Sensorspulen 41a, 41b werden über eine auf der Aufnahmeeinheit fixierte Leiterplatine 41c in Form einer als Flexprint bekannten gedruckten Leiterbahn auf einem formbaren oder flexiblen Träger kontaktiert. Die Leiterplatine 41c kann seitlich zwischen den zwei induktiven Sensorspulen 41a, 41b eine NFC (Near Field Communication) Spule zum Lesen von Informationen einer RFID (Radio Frequency IDentification) Etikette auf dem Injektionsgerät aufweisen. Eine flexible Verbindung 41d in Form eines Drahtes, Kabels, oder wie dargestellt als Fortsetzung der gedruckten Leiterbahnen des genannten Flexprint zur Übertragung von Signalen der Sensorspulen und der NFC Spule führt zu Leiterplatinen 64a, 64b der Elektronikeinheit. Die Flexibilität dieser Signalverbindung ermöglicht einen Ausgleich des variablen axialen Abstands zwischen Aufnahmeeinheit und Elektronikeinheit.

Die Elektronikeinheit 60 ist in einem proximalen Bereich des Innenraums unmittelbar an den Gehäuseabschluss 22 anschliessend angeordnet, und umfasst einen Elektronikhalter 61, einen Energiespeicher mit Batterie 62 und Akkumulator 63, eine erste und zweite Leiterplatine 64a, 64b mit Lichtquellen und Prozessoreinheiten zur Verarbeitung von Signalen der Sensorspulen 42a, 42b, zur Ansteuerung der Lichtquellen, und zur Kommunikation mit weiteren, externen Geräten. Die Elektronikeinheit umfasst weiter einen Lichtleiter 65 welcher das Licht der Lichtquellen an die Oberfläche des Modulgehäuses leitet, und eine Antenne 66 für einen Aufbau einer Kommunikationsverbindung mit einem Drittgerät, insbesondere für ein Out-of-Band Pairing zur Initiierung einer Bluetooth Verbindung.

Zwischen einer proximalen Endfläche der Aufnahmeeinheit und einer rechtwinklig zur Längsachse des Gehäuses ausgerichteten, distalen Endfläche des Elektronikhalters befindet sich ein elastisches Dämpfungselement 70 in Form einer komprimierten oder komprimierbaren Wendelfeder. Über die Endfläche spannt das Dämpfungselement 70 die Aufnahmeeinheit 40 in distaler Richtung vor. Das Dämpfungselement 70 kann auch zwischen anderen Komponenten des Modulgehäuses 20 und der Aufnahmeeinheit 40 oder dem Injektionsgerät axial wirkend vorgesehen sein.

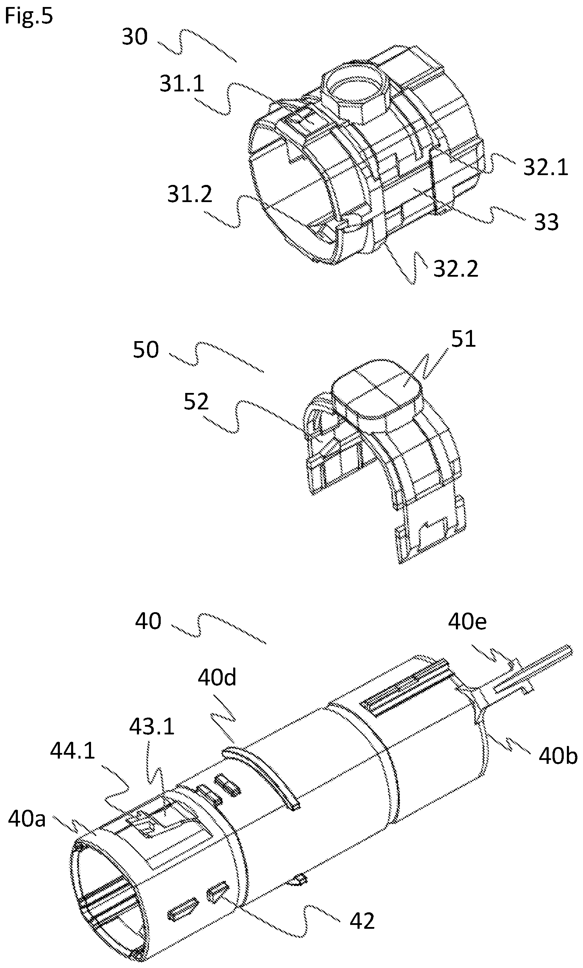

Fig.5 zeigt weitere Details von Gehäuseeinsatz, Aufnahmeeinheit, und Löseknopf in perspektivischer Ansicht. Der Gehäuseeinsatz 30 hat eine hülsenförmige Gestalt und weist in zwei gegenüberliegenden ersten Aussenseiten je eine Öffnung 31.1, 31.2 auf, welche in einem spitzen Winkel zur Längsachse des Gehäuseeinsatzes 30 von einer Aussenseite in proximaler Richtung durch die Wandung des Gehäuseeinsatzes 30 nach innen verlaufen. Der Gehäuseeinsatz 30 weist zudem in zwei weiteren, gegenüber den ersten Aussenseiten in Drehrichtung der Längsachse um 90° versetzten, zweiten Aussenseiten Durchbrüche 33 in der Wandung auf, durch welche Führungsnocken 52 des Löseknopfs 50 mit Sperrnocken 42 der Aufnahmeeinheit 40 zusammenwirken können. Weiter lagert der Gehäuseeinsatz 30 radial verschiebbar den Löseknopf 50. Hierzu umfasst der Gehäuseeinsatz 30 auf den beiden zweiten Aussenseiten je zwei Führungsnuten 32.1, 32.2. Der Gehäuseeinsatz 30 ist in einer radialen Vertiefung oder Ausbuchtung im Gehäuse 20 aufgenommen, wodurch der Gehäuseeinsatz 30 relativ zum Gehäuse 20 unbeweglich fixiert ist. In Umfangsrichtung ist der Gehäuseeinsatz 30 über auf Aussenseiten des Gehäuseeinsatzes 30 angebrachte Rippen, welche in entsprechende Nuten in der Innenwand des Gehäuses 20 eingreifen, relativ zum Gehäuse 20 unbeweglich gehalten.

Der Löseknopf 50 hat einen U-förmigen Querschnitt und ist auf dem Gehäuseeinsatz 30 gelagert, so dass die Schenkel der U-Form den Gehäuseeinsatz 30 beidseitig umgeben und in die Führungsnuten 32.1, 32.2 eingreifen. Zwischen den Schenkeln weist der Löseknopf 50 auf einer Aussenseite die Betätigungsfläche 51 auf, die der Benutzer mit der Hand betätigen kann, um den Löseknopf 50 radial in eine Richtung rechtwinklig zur Längsachse des Gehäuses 20 entlang einem linearen Sicherungsweg zu bewegen. Ferner weisen die Schenkel je auf ihrer Innenseite einen dreieckförmigen Führungsnocken 52 auf, welcher je mit einem dreieckförmigen Sperrnocken 42 der Aufnahmeeinheit 40 oder des Injektionsgerätes zusammenwirken kann und dadurch eine distale Bewegung der Aufnahmeeinheit 40 gegenüber dem Modulgehäuse 20 begrenzt. Zwischen dem Gehäuseeinsatz 30 und dem Löseknopf 50 befindet sich die Löseknopffeder 80, welche den Löseknopf 50 in eine Sicherungsrichtung radial nach aussen vorspannt.

Die Aufnahmeeinheit 40 ist in axialer Richtung relativ zum Gehäuse 20 und damit relativ zum Gehäuseeinsatz 30 bewegbar gelagert. Die Aufnahmeeinheit 40 ist ebenfalls hülsenförmig ausgeführt und hat im Querschnitt im Wesentlichen eine an den Querschnitt des Autoinjektors angepasste quadratische Form, wobei die Seitenwände des Quadrats jeweils leicht nach aussen gewölbt sind. Weiter umfasst die Aufnahmeeinheit 40 zur proximalen Begrenzung des Innenraums eine Endfläche 40b, die rechtwinklig zur Längsachse der Aufnahmeeinheit 40 ausgerichtet ist und sich über den ganzen Querschnitt des Innenraums der Aufnahmeeinheit 40 erstreckt.

Auf zwei einander gegenüberliegenden ersten Seiten der nahezu quadratischen Form der Aufnahmeeinheit 40 ist je ein Halteelement in der Form eines Haltearms 43.1, 43.2 angeordnet, wobei die Haltearme 43.1, 43.2 sich in Bezug auf die axiale Länge der Aufnahmeeinheit 40 in einem distalen Bereich der Aufnahmeeinheit 40 befinden. Die Haltearme 43.1. 43.2 sind an einem ersten Ende schwenkbar mit der Aufnahmeeinheit 40 verbunden. Im Bereich des freien Endes auf einer dem Innenraum abgewandten Aussenseite der Haltearme 43.1, 43.2 weisen diese je eine Auskragung 44.1, 44.2 auf, welche je in den Öffnungen 31.1, 31.2 aufgenommen werden können. Ebenfalls im Bereich des freien Endes der Haltearme 43.1, 43.2 jedoch auf einer Innenseite, welche dem Innenraum der Aufnahmeeinheit 40 zugewandt ist, weisen die Haltearme 43.1, 43.2 je einen Vorsprung 45.1, 45.2 auf. Auf den anderen zwei Seiten der quadratischen Form ist je ein dreieckförmiger Sperrnocken 42 angeordnet, welcher jeweils mit einem Führungsnocken 52 des Löseknopfs 50 zusammenwirken kann.

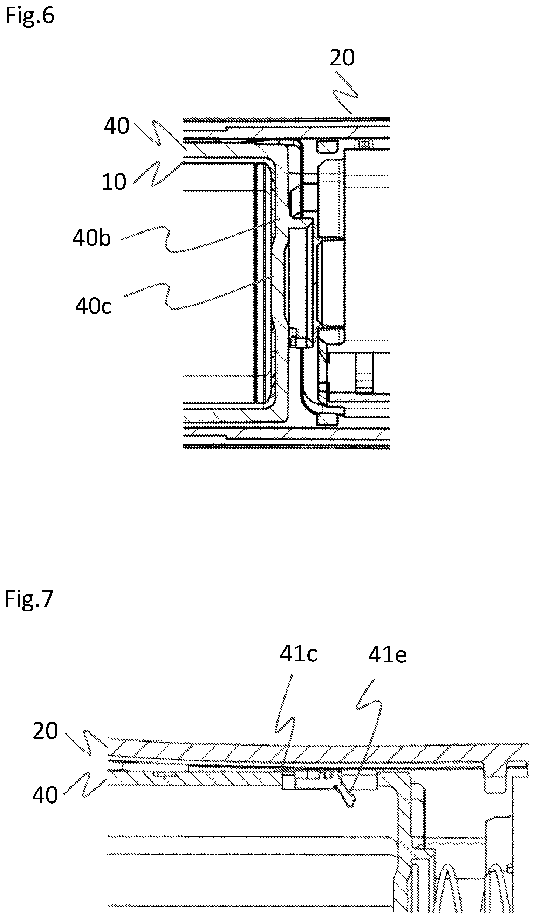

Fig.6 zeigt einen Ausschnitt im Bereich der Endfläche 40b eines Längsschnitts durch Zusatzmodul und Injektionsgerät. Die distale Seite der Endfläche 40b weist in der Mitte eine Ausbuchtung 40c oder eine Erhöhung auf mit einem ebenen Plateau auf welchem eine proximale Stirnfläche des Injektionsgerätes aufliegt. Die Endfläche 40b kann aber auch mit diskreten Elementen zur nichtflächigen Kontaktierung und Kraftübertragung auf das Injektionsgerät versehen sein, beispielsweise mit einer zentralen konvexen Wölbung, mit mehreren über den Querschnitt verteilten Stiften oder Zapfen, oder mit eindimensionalen Stegen, welche bevorzugt konzentrisch zum Querschnitt des Autoinjektors angeordnet sind. Andererseits kann die Form der Endfläche 40b an eine unebene Form der proximalen Stirnfläche des Injektionsgerätes angepasst sein, um einen grossflächigen Kontakt zu ermöglichen.

In der perspektivischen Ansicht aus Fig.5 sind weiter dargestellt ein Flanschabschnitt 40d welcher sich in radialer Richtung nach aussen erstreckt, sowie ein in proximale Richtung weisender Arm 40e welcher zur Vorverschnappung bei der Montage der Aufnahmeeinheit in das Gehäuse dient. Eine Verlängerung oder Spitze auf dem Arm 40e wirkt auf einen nicht dargestellten mechanischen oder optischen Schalter und aktiviert die Prozessoreinheiten aus einem Standby- oder Ruhezustand sobald die Aufnahmeeinheit um einen Aufnahmeweg nach proximal verschoben wird. Der auf einer Leiterplatine 64 angeordnete Schalter detektiert also das Ende der Aufnahmebewegung von Arm 40e und damit der Aufnahmeeinheit 40, ein allfälliges Fehlen des Injektionsgerätes kann anschliessend über die aktivierten Sensorelemente (41a, 41b) oder ein sonstiges RF-Identifikationssignal des Injektionsgerätes festgestellt werden. An Stelle der Leiterplatine 64 kann ein Schalter zur Detektion der axialen Position der Aufnahmeeinheit auch an einer beliebigen Stelle auf der Innenseite des Gehäuses 20 montiert sein, und dort mit einem Vorsprung, beispielsweise dem genannten Flanschabschnitt 40d, oder einer Nut der Aufnahmeeinheit zusammenwirken.

Alternativ kann ein Schalter zur Detektion der axialen Position der Aufnahmeeinheit auf der Aufnahmeeinheit selbst angeordnet sein und mit einem gehäusefesten Element oder Vorsprung zusammenwirken. In diesem Falle kann der Schalter bestückt sein auf der als Flexprint gefertigten gedruckten Leiterplatine 41c, welche dauerhaft auf der Aussenfläche der Aufnahmeeinheit befestigt ist. In einer Ausführungsform mit einem Löseknopf 50, welcher bei Einführung des Injektionsgeräts gegenüber dem Gehäuse 20 und/oder Gehäuseeinsatz 30 bewegt wird, kann auch diese Relativbewegung durch einen Schalter detektiert werden. Auch in allen diesen Fällen dient ein mechanischer Schalter primär zur Aktivierung der Elektronik und weiterer Sensorelemente, so dass die axiale Positionierung des Schalters nicht kritisch ist und der Schalter schon vor Erreichen der Haltekonfiguration auslösen kann.

Wird ein Schalter auf der Leiterplatine 64 angeordnet und mit einem geeignet ausgedehnten Stössel versehen oder mit einem separat gefederten Adapter ergänzt, wobei Stössel und Adapter bevorzugt die Endfläche 40b in Längsrichtung durchdringen, kann auch unmittelbar die Anwesenheit des Autoinjektors in der Haltekonfiguration festgestellt werden. In diesem Fall kann auf Sensorelemente zur zusätzlichen Identifikation des Injektionsgerätes verzichtet werden.

Fig.7 zeigt einen Ausschnitt aus einem Längsschnitt durch das Zusatzmodul mit einem Schalter 41e zur Detektion der Anwesenheit des Injektionsgerätes in der Aufnahmeeinheit 40. Der Schalter ist seitlich auf der Aufnahmeeinheit angeordnet, bevorzugt auf der als Flexprint gefertigten gedruckten Leiterplatine 41c neben einer seitlichen Öffnung in der Aufnahmeeinheit. In diesem Fall muss die Anwesenheit der Aufnahmeeinheit inklusive Injektionsgerät in der Haltekonfiguration zusätzlich festgestellt werden.

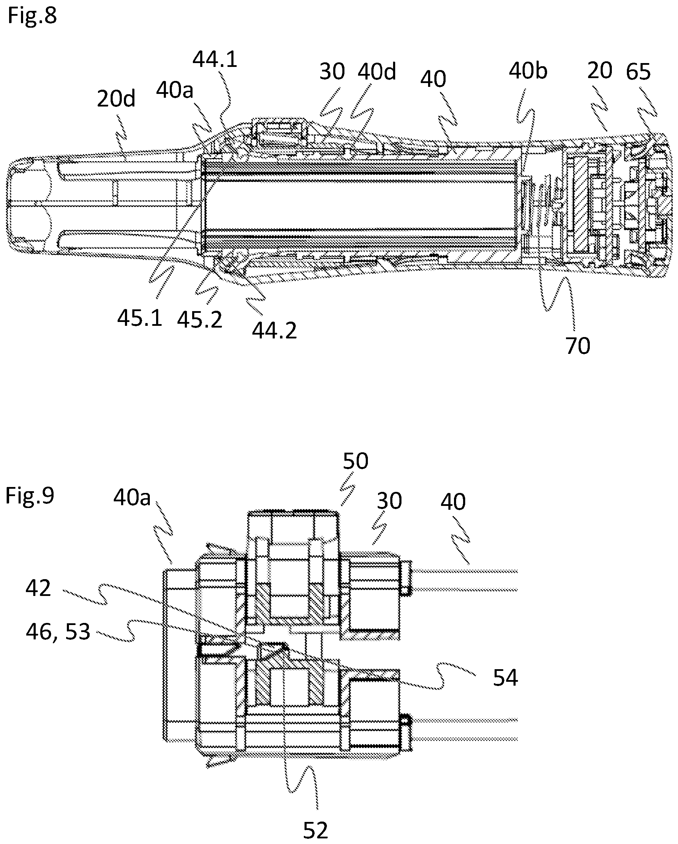

In Fig.8 ist eine Schnittansicht des Zusatzmoduls 2 in einer Freigabekonfiguration ersichtlich, wobei der Schnitt durch die Längsachse des Zusatzmoduls 2 verläuft. Die im Modulgehäuse 20 verschiebbar gelagerte Aufnahmeeinheit 40 befindet sich in der Freigabekonfiguration an einem distalen Ende eines Aufnahme- oder Konfigurationsverstellwegs. Die Aufnahmeeinheit 40 wird durch eine Aufnahmefeder als elastisches Dämpfungselement 70 in diese Position gedrängt und stösst dabei mit einem Flanschabschnitt 40d (eingekreist) oder einem radialen Pin an einer durch den Gehäuseeinsatz 30 gebildeten distalen Aufnahmewegbegrenzung an. Die Anzeigekante 40a ragt dabei in den Sichtbereich der Längsöffnung 20d, ist für einen Nutzer somit sichtbar und zeigt ihm dadurch die Freigabestellung an. Wenn die Aufnahmeeinheit 40 in proximaler Richtung relativ zum Gehäuse 20 verschoben werden soll, muss sie gegen die Vorspannkraft der Aufnahmefeder bewegt werden. Die Aufnahmeeinheit 40 kann in proximaler Richtung relativ zum Gehäuse 20 verschoben werden bis die Aufnahmefeder 70 maximal komprimiert ist oder bis der Flanschabschnitt 40d an einer durch das Gehäuse gebildeten proximalen Aufnahmewegbegrenzung anstösst. Letztere kann gleichzeitig als Anschlag fungieren und mit dem Flanschabschnitt 40d als Verzögerungsendanschlag zusammenwirken.

In Fig.8 ist zudem ersichtlich, dass die beiden Haltearme 43.1, 43.2 vom Körper der Aufnahmeeinheit 40 nach aussen abstehen, indem jeweils die Auskragung 44.1, 44.2 der Haltearme 43.1, 43.2 in den Öffnungen 31.1, 31.2 im Gehäuseeinsatz 30 aufgenommen und in diesen zwangsgeführt sind. Da die Haltearme 43.1, 43.2 dadurch nach aussen weisen, ragen die Vorsprünge 45.1, 45.2 (eingekreist) auf den jeweiligen Innenseiten der Haltearme 43.1, 43.2 nicht in den Innenraum der Aufnahmeeinheit 40, sondern sind im Wesentlichen bündig mit der Innenfläche der Aufnahmeeinheit 40. In dieser Stellung befinden sich die Haltearme 43.1, 43.2 also in einer Freigabeposition.

Fig.9 zeigt gegenüber Fig.8 vergrössert einen mittleren Bereich des Zusatzmoduls 2 in einer Freigabekonfiguration, wobei die äussere Wandung des Gehäuses 20 und des Gehäuseeinsatzes 30 und ein Schenkel der U-Form des Löseknopfs 50 in der Darstellung weggeschnitten sind. Wie oben erwähnt, weist die Aufnahmeeinheit 40 auf zwei Aussenseiten je einen Sperrnocken 42 auf, und der Löseknopf 50 umfasst auf seiner Innenseite zwei einander radial gegenüberliegende Führungsnocken 52, wobei einer der Führungsnocken 52 und einer der Sperrnocken 42 in Fig.9 ersichtlich sind.

In einer Freigabekonfiguration befinden sich die Sperrnocken 42 auf der distalen Seite der Führungsnocken 52. Die Sperrnocken 42 umfassen je eine Kontaktfläche 46, welche in einem spitzen Winkel zur Längsachse des Gehäuses 20 steht. Die Führungsnocken 52 umfassen je eine Führungsfläche 53, die ebenfalls in diesem Winkel zur Längsachse ausgerichtet sind. In der gezeigten Freigabekonfiguration befinden sich die Führungsnocken 52 in einer Lösestellung, in der die Kontaktflächen 46 der Sperrnocken 42 und die Führungsflächen 53 der Führungsnocken 52 aneinander liegen. Die Führungsfläche 53 ist in Richtung der Kontaktfläche 46 durch die Löseknopffeder 80 vorgespannt. In einer Freigabekonfiguration, in der sich die Aufnahmeeinheit 40 an einem durch die relative Anordnung von Flanschabschnitt 40d und distaler Aufnahmewegbegrenzung bestimmten distalen Ende ihres Aufnahmewegs befindet, können die Führungsfläche 53 und die Kontaktfläche auch axial beabstandet sein.

Zum Verbinden des Injektionsgeräts 1 mit dem Zusatzmodul 2 muss Letzteres von der Freigabekonfiguration in eine Haltekonfiguration verstellt werden. Hierzu muss die Aufnahmeeinheit 40 relativ zum Gehäuse 20 von einer distalen in eine proximale Endposition verschoben werden. Diese Verschiebung erfolgt durch das Injektionsgerät 1, dessen proximalen Ende in die Öffnung am distalen Ende des Gehäuses 20 eingeschoben und in Richtung proximales Ende des Gehäuses 20 bewegt wird. Hierbei trifft zuerst das proximale Ende des Injektionsgeräts 1 auf die Endfläche 40b der Aufnahmeeinheit 40, anschliessend werden das Injektionsgerät und die Aufnahmeeinheit 40 gegen die Aufnahmefeder in proximaler Richtung verschoben. Bei dieser Verschiebung werden die Haltearme 43.1, 43.2 in proximaler Richtung mitbewegt, wodurch die Auskragungen 44.1, 44.2 der Haltearme 43.1, 43.2 jeweils aus den schrägen Öffnungen 31.1, 31.2 im Gehäuseeinsatz 30 herausgleiten und sich zum Innenraum des Gehäuses beziehungsweise zum eingesetzten Injektionsgerät 1 hinbewegen. Dadurch bewegen sich jeweils auch die Vorsprünge 45.1, 45.2 an den Innenseiten der Haltearme 43.1, 43.2 zum Injektionsgerät 1 hin und greifen mit zunehmender Verschiebung der Aufnahmeeinheit 40 in proximaler Richtung in Aussparungen 10b im Injektionsgerät 1 ein. Durch fortgesetztes Verschieben der Aufnahmeeinheit 40 werden die Auskragungen 44.1, 44.2 weiter durch die Innenwandung des Gehäuseeinsatzes 30 geführt, so dass die Haltearme 43.1, 43.2 nicht mehr in radialer Richtung nach aussen gelenkt werden können. Die Haltearme 43.1, 43.2 sind somit durch den Gehäuseeinsatz 30 in einer Halteposition verriegelt und die Vorsprünge 45.1, 45.2 können sich nicht unbeabsichtigt aus den Aussparungen 10b im Injektionsgerät 1 lösen.

Bei der Verschiebung der Aufnahmeeinheit von der distalen Endposition in die proximale Endposition wirken auch die Sperrnocken 42 der Aufnahmeeinheit 40 mit den Führungsnocken 52 des Löseknopfs 50 zusammen. Sobald nämlich die Aufnahmeeinheit 40 von ihrer distalen Endposition in proximaler Richtung verschoben wird, drücken jeweils die schrägen Kontaktflächen 46 der Sperrnocken 42 der Aufnahmeeinheit 40 gegen die schrägen Führungsflächen 53 der Führungsnocken 52 des Löseknopfs 50. Da sich der Löseknopf 50 nicht in axialer Richtung bewegen kann, jedoch entgegen der Sicherungsrichtung in radialer Richtung bewegbar gelagert ist, verschieben sich die Führungsnocken 52 durch den Druck der Sperrnocken 42 entgegen der Vorspannkraft der Löseknopffeder 80 in radialer Richtung, bis die Sperrnocken 42 über die Führungsflächen 53 hinausgleiten und dadurch die Führungsnocken 52 freigeben. In diesem Moment springt der Löseknopf 50 durch die Federkraft der Löseknopffeder 80 wieder in Sicherungsrichtung. Die Sperrnocken 42 befinden sich dann auf der proximalen Seite der Führungsnocken 52. Die Führungsnocken 52 befinden sich in dieser Position in ihrer Haltestellung, in der sie mit einer rechtwinklig zur Längsachse ausgerichteten Verriegelungsfläche 54 die Sperrnocken 42 an einer Bewegung in distaler Richtung hindern. In dieser Position befindet sich die Aufnahmeeinheit 40 an einem proximalen Ende ihres Aufnahmewegs und das Zusatzmodul 2 befindet sich in der Haltekonfiguration.

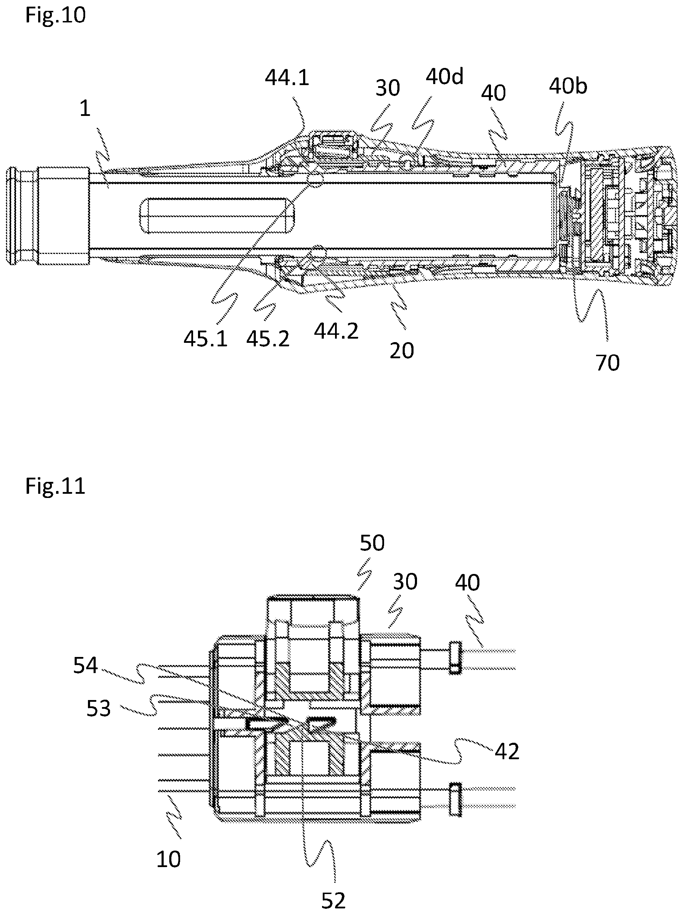

Fig.10 zeigt das Zusatzmodul 2 in einer Schnittansicht in der Haltekonfiguration mit einem im Gehäuse 20 gehaltenen Injektionsgerät 1 in Aufsicht. Dieses berührt mit seiner Stirnfläche die Endfläche 40b der Aufnahmeeinheit 40, welche sich am proximalen Ende des Aufnahmewegs befindet. Die Aufnahmefeder ist in dieser Position der Aufnahmeeinheit 40 noch nicht maximal komprimiert, der Flanschabschnitt 40d (eingekreist) befindet sich zwischen der distalen Aufnahmewegbegrenzung und einer nach distal gerichteten Anschlagsfläche im Innern des Modulgehäuses. Durch das oben beschriebene Zusammenwirken von Sperrnocken 42 und Führungsnocken 52 wird die Aufnahmeeinheit 40 in dieser durch die Aufnahmefeder vorgespannten Position gehalten. Die Haltearme 43.1, 43.2 befinden sich in ihrer Halteposition, in der jeweils die Vorsprünge 45.1, 45.2 in die Aussparungen 10b im Injektionsgerät 1 eingreifen (Position der Vorsprünge und Aussparungen ist eingekreist, aber durch die Aufsicht des Injektionsgerätes verdeckt) und in der die Innenwandung die Auskragungen 44.1, 44.2 der Haltearme 43.1, 43.2 gegen radiale Bewegung verriegelt.

Fig.11 zeigt einen mittleren Abschnitt des Zusatzmoduls in der Haltekonfiguration. Dabei ist wie in Fig.9 eine äussere Wandung des Gehäuses 20 und des Gehäuseeinsatzes 30, sowie ein Teil des Schenkels des Löseknopfs 50 weggeschnitten, damit einer der Führungsnocken 52 des Löseknopfs 50 und einer der Sperrnocken 42 der Aufnahmeeinheit 40 ersichtlich sind. In Fig.11 ist der Führungsnocken 52 in seiner Haltestellung gezeigt. In dieser hindert der Führungsnocken 52 mit seiner Verriegelungsfläche 54 und durch die mit der Löseknopffeder 80 erzeugten Vorspannkraft des Löseknopfs 50 den Sperrnocken 42 und damit die Aufnahmeeinheit 40 an einer Bewegung in eine distale Richtung. Somit ist ein unerwünschtes Verschieben der Aufnahmeeinheit 40 zumindest in distale Richtung nicht möglich und der Injektor 1 kann sich nicht unbeabsichtigt aus dem Gehäuse 20 des Zusatzmoduls 2 lösen.

Um das Injektionsgerät 1 vom Zusatzmodul 2 zu trennen, muss das Zusatzmodul 2 von der Haltekonfiguration in die Freigabekonfiguration verstellt werden. Dies wird erreicht, indem die Betätigungsfläche 51 des Löseknopfs 50 gedrückt wird, so dass sich der Löseknopf entlang seines Sicherungswegs in radialer Richtung zum Gehäuse 20 hin verschiebt. Dadurch wird die Löseknopffeder 80 komprimiert und die Führungsnocken 52 verschieben sich von ihrer Haltestellung in eine Freigabestellung, wodurch die Sperrnocken 42 der Aufnahmeeinheit 40 freigegeben werden und in distaler Richtung bewegt werden können.

Durch manuelles Drücken des Löseknopfs 50 gegen die Sicherungsrichtung und quer zur Längsrichtung wird also eine kurze, aber dennoch gut erkennbare Freigabe- oder Auswurfbewegung der Aufnahmeeinheit 40 in distaler Richtung des Aufnahmewegs gestartet, an deren Ende das Zusatzmodul sich in der Freigabekonfiguration befindet und die Entfernung des Injektionsgeräts aus dem Zusatzmodul erfolgen kann. Die Auswurfbewegung der Aufnahmeeinheit kann dabei ausschliesslich durch die unmittelbar aufgewendete Kraft des Benutzers erfolgen, beispielsweise durch geschickte Umlenkung der Kraft quer zur Längsrichtung über eine Kurvensteuerung oder einen Keil in eine Kraft in Richtung des Aufnahmewegs, oder auch durch Drücken eines alternativen Löseknopfs oder manuelles Verschieben der Aufnahmeeinheit in Längsrichtung. Die Auswurfbewegung kann aber auch vollständig automatisch ablaufen und von einer vorgefüllten Energiequelle getrieben werden, so dass der Löseknopf ausschliesslich als Trigger dient und somit auch als elektronischer Berührungssensor ausgeführt werden kann. Vorgefüllte Energiequellen sind bei Auslieferung des Zusatzmoduls maximal geladenen und geben bei jedem Auswurf eine kleine Energiemenge ab. Neben einem vorgespannten mechanischen Speicher in Form einer Feder oder einem Druckgasreservoir ist auch ein elektrischer Antrieb denkbar.

Die Freigabe- oder Auswurfbewegung kann auch unterstützt werden durch Abgabe von Energie aus einer wieder aufladbaren Energiequelle als Freigabeunterstützung, welche im Moment des axialen Einschubs des Injektionsgeräts in das Zusatzmodul geladen wird. Zu diesem Zweck kann ein separates Federelement, ein komprimierbares Gasvolumen, oder auch die Aufnahmefeder als elastisches Dämpfungselement 70 vorgesehen sein. Beim Auswurf kann sich das in der Haltekonfiguration komprimierte Federelement entspannen und auf die Endfläche 40b drücken, wodurch die Aufnahmeeinheit 40 in distaler Richtung verschoben wird. Das Laden oder Spannen der Freigabeunterstützung kann aber auch erst im Moment des Auswurfs durch initiales Drücken des Löseknopfs 50 über einen ersten Teil-hub geschehen, wobei eine anschliessende finale Bewegung des Löseknopfs über einen zweiten Teil-hub als Trigger für die Unterstützung dient.

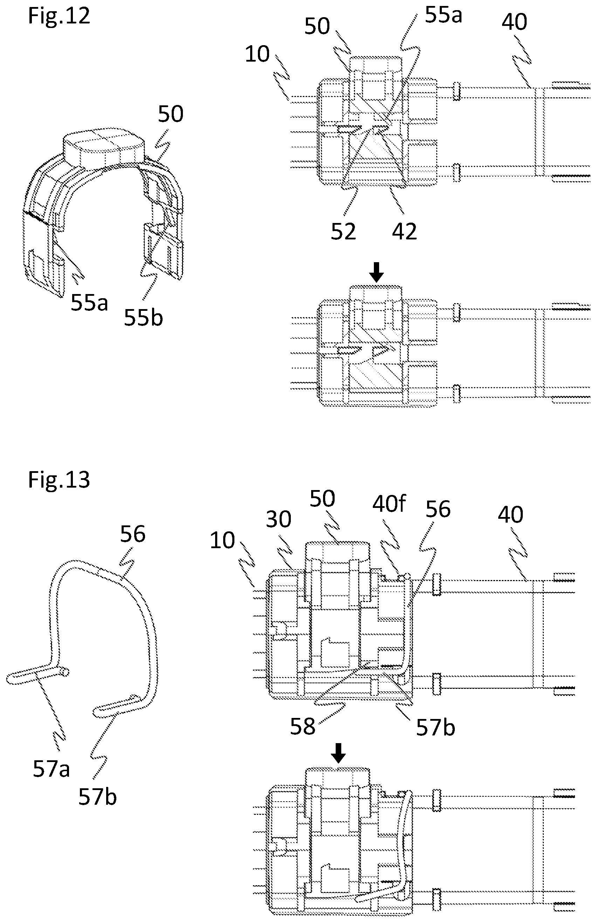

Fig.12 zeigt eine erste Variante mit einer spannbaren Freigabeunterstützung umfassend zwei elastische Federarme 55a, 55b oder Laschen, welche auf je einer Innenseite der beiden Schenkel des Löseknopfs 50 befestigt und bevorzugt einstückig mit diesem ausgebildet sind. In der in Fig.12 rechts oben dargestellten Haltekonfiguration ist ein Teil eines Schenkels weggelassen, so dass ein Federarm 55a, ein Sperrnocken 42, und ein Führungsnocken 52 sichtbar sind. Ein proximales Ende des Sperrnockens 42 liegt leicht distal verschoben neben dem ungespannten Federarm 55a. Bei einer Bewegung des Löseknopfs 50 mit Federarm 55 und Führungsnocken 52 entgegen seines Sicherungswegs in Pfeilrichtung wird der Federarm am Sperrnocken 42 gespannt. Wie in Fig. 12 rechts unten erkennbar gibt gegen Ende dieser Bewegung der Führungsnocken 52 den Sperrnocken 42 für eine Bewegung in distaler Richtung frei, und die vorgängig im Federarm 55a gespeicherte Energie treibt den Sperrnocken 42 und damit die Aufnahmeeinheit 40 nach distal. An Stelle des Sperrnockens kann der Federarm auch auf einen anderen Vorsprung der Aufnahmeeinheit wirken.

Fig.13 zeigt eine zweite Variante mit einer spannbaren separaten Freigabeunterstützung aus Federdraht in Form einer Bügelfeder mit einem U-förmigen Bügel 56 und zwei rechtwinklig davon abgewinkelten Spannschenkeln 57a, 57b. An den Übergängen zwischen Bügel und Spannschenkel befinden sich zwei Dreh- und Auflagepunkte, an welchen die Bügelfeder am Gehäuseeinsatz 30 eingespannt ist. In der in Fig.13 rechts oben dargestellten Haltekonfiguration sind die Spannschenkel 57 ungefähr parallel zur Längsachse ausgerichtet, so dass ein distales Ende der Spannschenkel in Sicherungsrichtung unterhalb einer Kante 58, einer Nase, oder einem Vorsprung des Löseknopfs positioniert ist. Der Bügel 56 steht an einem Pin 40f der Aufnahmeeinheit gegen distal an, oder ist in einer Ausnehmung der Aufnahmeeinheit geführt. Wie in Fig.13 rechts unten dargestellt werden durch eine Bewegung des Löseknopfs in Pfeilrichtung während einem ersten Teil-hub die Spannschenkel nach unten gedrückt und der Bügel bei zumindest annähernd starrem Winkel zwischen Spannschenkel und Bügel gespannt. Eine Freigabe der Aufnahmeeinheit erfolgt anschliessend wie in der vorgängig beschriebenen Variante.

Durch die Bewegung der Aufnahmeeinheit in distaler Richtung werden die Haltearme 43.1, 43.2 ebenfalls in distaler Richtung verschoben, und die Auskragungen 44.1, 44.2 führen entlang der Innenwandung des Gehäuseeinsatzes 30 und formbedingt in die Öffnungen 31.1, 31.2 hinein und werden durch Letztere zwangsgeführt. Die Haltearme 43.1, 43.2 werden schräg nach aussen vom Innenraum der Aufnahmeeinheit 40 wegbewegt, wodurch auch die Vorsprünge 45.1, 45.2 vom Innenraum beziehungsweise vom Injektionsgerät 1 radial weg und aus dessen Aussparung 10b herausbewegt werden. Durch die Kraft der Aufnahmefeder wird die Aufnahmeeinheit 40 bis zum distalen Ende ihres Aufnahmewegs beziehungsweise einem distalen Anschlag am Gehäuse 20 verschoben. Während dieser Bewegung der Aufnahmeeinheit 40 werden die freien Ende der Haltearme 43.1, 43.2 weiter in die jeweiligen Öffnungen 31.1, 31.2 im Gehäuseeinsatz 30 hineingeschoben bis sich die Haltearme 43.1. 43.1 in ihrer Freigabeposition befinden. Wenn die Aufnahmeeinheit 40 am distalen Ende ihres Aufnahmewegs angelangt ist, befindet sich das Zusatzmodul in der Freigabekonfiguration und der Injektor 1 kann aus dem Gehäuse 20 entnommen werden.

**BEZUGSZEICHENLISTE**

| | | | |
|---|---|---|---|
| 1 | Injektionsgerät | 41a, 41b | Sensorspulen |
| 10 | Gerätegehäuse | 41c | Leiterplatine |
| 10a | Fenster | 41d | Verbindung |
| 10b | Aussparung | 41e | Schalter |
| 11 | Nadelschutzhülse | 42 | Sperrnocken |
| 12 | Nadelschutzkappenentferner | 43.1, 43.2 | Haltearm |
| 2 | Zusatzmodul | 44.1, 44.2 | Auskragung |
| 20 | Modulgehäuse | 45.1, 45.2 | Vorsprung |
| 20a | Griffposition | 46 | Kontaktfläche |
| 20b | Lasche | 50 | Löseknopf |
| 20c | Quersteg | 51 | Betätigungsfläche |
| 20d | Längsöffnung | 52 | Führungsnocken |
| 21a, 21b | Gehäusehälften | 53 | Führungsfläche |
| 22 | Gehäuseabschluss | 54 | Verriegelungsfläche |
| 23 | Radialöffnung | 55a, 55b | Federarm |
| 24 | Anschlag | 56 | Bügel |
| 30 | Gehäuseeinsatz | 57a, 57b | Spannschenkel |
| 31.1, 31.2 | Öffnung | 58 | Nase |
| 32.1, 32.2 | Führungsnuten | 60 | Elektronikeinheit |
| 33 | Durchbruch | 61 | Elektronikhalter |
| 40 | Aufnahmeeinheit | 62 | Batterie |
| 40a | Anzeigekante | 63 | Akkumulator |
| 40b | Endfläche | 64a, 64b | Leiterplatine |
| 40c | Ausbuchtung | 65 | Lichtleiter |
| 40d | Flanschabschnitt | 66 | Antenne |
| 40e | Arm | 70 | Dämpfungselement |
| 40f | Pin | 80 | Löseknopffeder |

## Patentansprüche

1. Elektronisches Zusatzmodul (2) zum Aufsetzen vor Injektionsbeginn auf ein Injektionsgerät (1) mit einer Längsachse, welche ein einstechseitiges Ende mit einem gegenüberliegenden proximalen Ende des Injektionsgeräts verbindet, umfassend ein Sensorelement (41a, 41b) zur Detektion eines Zustandes des Injektionsgeräts, ein Prozessorelement (64a, 64b) zur Auswertung eines Signals des Sensorelements, und einen Energiespeicher (62, 63) zur Energieversorgung des Prozessorelementes, wobei das Zusatzmodul (2) weiter umfasst
- ein erstes Modulteil (40), welches einen Haltemechanismus umfasst, welcher ein Haltearm (43.1, 43.2) umfasst, wobei das erste Modulteil (40) mithilfe des Haltearms mit einem Vorsprung (44.1, 44.2) zum Eingreifen in eine Aussparung des Injektionsgerätes in Richtung der Längsachse axialfest mit dem Injektionsgerät verbindbar ist, und
- ein zweites Modulteil (20, 30, 50, 60),
**dadurch gekennzeichnet, dass** das zweite Modulteil in einer distalen Richtung der Längsachse für eine Relativbewegung gegenüber dem axialfest mit dem Injektionsgerät verbundenen ersten Modulteil parallel zur Längsachse beweglich ist wobei, der Haltemechanismus unabhängig von dieser Relativbewegung bis zur Betätigung einer Löseeinheit (50) wirksam bleibt, so dass das erste Modulteil während der Relativbewegung axialfest mit dem Injektionsgerät verbunden bleibt und diese Relativbewegung nicht zu einer Lösung des Zusatzmoduls (2) vom Injektionsgerät (1) führt, sowie durch
- ein Dämpfungselement (70) zur Dämpfung der Relativbewegung.

2. Zusatzmodul nach Anspruch 1, **dadurch gekennzeichnet, dass** das Dämpfungselement (70) unmittelbar zwischen den beiden Modulteilen angeordnet ist.

3. Zusatzmodul nach Anspruch 1, **dadurch gekennzeichnet, dass** das zweite Modulteil eine grössere Masse aufweist als das erste Modulteil.

4. Zusatzmodul nach Anspruch 1, **dadurch gekennzeichnet, dass** das Sensorelement (41a, 41b) im ersten Modulteil angeordnet ist.

5. Zusatzmodul nach Anspruch 2, **dadurch gekennzeichnet, dass** das zweite Modulteil einen Anschlag (24) aufweist, welcher am Ende der gedämpften Relativbewegung auf einen Verzögerungsendanschlag des Injektionsgerätes oder des ersten Modulteils aufschlagen kann.

6. Zusatzmodul nach Anspruch 2, **dadurch gekennzeichnet, dass** das Dämpfungselement (70) ein Federelement umfasst, welches beim Verbinden des Zusatzmoduls mit dem Injektionsgerät vorgespannt wird.

7. Zusatzmodul nach einem der Ansprüche 1 bis 6, **dadurch gekennzeichnet, dass** das erste Modulteil einen Innenraum aufweist und dass das zweite Modulteil die gedämpfte Relativbewegung innerhalb dieses Innenraums ausführt.

8. Zusatzmodul nach einem der Ansprüche 1 bis 6, **dadurch gekennzeichnet, dass** das erste Modulteil eine Aufnahmeeinheit (40) und das zweite Modulteil ein Modulgehäuse (20) umfasst, welches die Aufnahmeeinheit umgibt.

9. Zusatzmodul nach Anspruch 8, **dadurch gekennzeichnet, dass** das zweite Modulteil einen Griff zur Ergreifung von Injektionsgerät und Zusatzmodul aufweist.

10. Zusatzmodul nach Anspruch 5 und nach Anspruch 8 oder 9, **dadurch gekennzeichnet, dass** der Anschlag (24) des zweiten Modulteils am Ende der gedämpften Relativbewegung der Modulteile auf einen Verzögerungsendanschlag eines Nadelschutzkappenentferners (12) des Injektionsgeräts aufschlagen kann.

11. Zusatzmodul nach einem der Ansprüche 8 bis 10, **dadurch gekennzeichnet, dass** das das zweite Modulteil die Löseeinheit (50) umfasst, wobei
das Zusatzmodul (2) in einer Haltekonfiguration durch die Löseeinheit (50) eine distale Bewegung der Aufnahmeeinheit (40) im Modulgehäuse (20) begrenzt und der Haltearm (43.1, 43.2) das Injektionsgerät (1) in der Aufnahmeeinheit (40) hält;
das Zusatzmodul (2) in einer Freigabekonfiguration die Aufnahmeeinheit (40) von der Löseeinheit (50) und das Injektionsgerät (1) vom Haltearm (43.1, 43.2) freigibt,
wobei das Zusatzmodul (2) durch eine Freigabebewegung der Aufnahmeeinheit (40) von der Haltekonfiguration in die Freigabekonfiguration verstellbar ist.

12. Zusatzmodul (2) nach Anspruch 11, **dadurch gekennzeichnet, dass** die Löseeinheit (50) einen Führungsnocken (52) und die Aufnahmeeinheit (40) oder das Injektionsgerät (1) einen Sperrnocken (42) umfasst, wobei in der Haltekonfiguration der Führungsnocken (52) eine Haltestellung einnimmt und derart mit dem Sperrnocken (42) zusammenwirkt, dass die Aufnahmeeinheit (40) und das Injektionsgerät (1) an einer Bewegung in distaler Richtung gehindert sind.

13. Zusatzmodul (2) nach Anspruch 12, **dadurch gekennzeichnet, dass** der Führungsnocken (52) von der Haltestellung in eine Freigabestellung, in welcher der Sperrnocken (42) vom Führungsnocken (52) freigegeben ist, verstellbar ist und wobei der Führungsnocken (52) durch eine Löseknopffeder (80) in seine Haltestellung vorspannbar ist.

14. Zusatzmodul nach einem der Ansprüche 8 bis 13, **dadurch gekennzeichnet, dass** das Zusatzmodul eine Freigabeunterstützung (55, 56) umfasst, welche beim Betätigen einer oder der Löseeinheit (50) zuerst gespannt wird und sich anschliessend unter Verschiebung der Aufnahmeeinheit (40) gegenüber dem Modulgehäuse (20) entspannt.

15. Injektionsgerät (1) mit einem Zusatzmodul (2) nach einem der Ansprüche 1 bis 14, **dadurch gekennzeichnet, dass** das erste Modulteil zwei Haltearme (43.1, 43.2) umfasst, welche so ausgebildet sind, dass sie formschlüssig in Aussparungen im Injektionsgerät eingreifen können, um die axialfeste Verbindung zwischen Injektionsgerät (1) und erstem Modulteil herzustellen.

## Claims

1. Electronic add-on module (2) for placement, prior to the start of injection, on an injection device (1) having a longitudinal axis which connects an injection-side end to an opposite proximal end of the injection device, comprising a sensor element (41a, 41b) for detecting a state of the injection device, a processor element (64a, 64b) for evaluating a signal from the sensor element, and an energy store (62, 63) for supplying energy to the processor element, the add-on module (2) further comprising
- a first module part (40) comprising a holding mechanism which comprises a holding arm (43.1, 43.2), it being possible for the first module part (40) to be axially fixedly connected to the injection device in the direction of the longitudinal axis using the holding arm having a projection (44.1, 44.2) for engaging in a recess of the injection device, and
- a second module part (20, 30, 50, 60),
**characterized in that** the second module part is movable in a distal direction of the longitudinal axis for a relative movement with respect to the first module part, which is axially fixed to the injection device, in parallel with the longitudinal axis, the holding mechanism remaining effective independently of this relative movement until a release unit (50) is actuated so that the first module part remains axially fixedly connected to the injection device during the relative movement and this relative movement does not lead to the add-on module (2) being released from the injection device (1), and **by**
- a damping element (70) for damping the relative movement.

2. Add-on module according to claim 1, **characterized in that** the damping element (70) is arranged directly between the two module parts.

3. Add-on module according to claim 1, **characterized in that** the second module part has a larger mass than the first module part.

4. Add-on module according to claim 1, **characterized in that** the sensor element (41a, 41b) is arranged in the first module part.

5. Add-on module according to claim 2, **characterized in that** the second module part has a stop (24) which can strike a delay end stop of the injection device or of the first module part at the end of the damped relative movement.

6. Add-on module according to claim 2, **characterized in that** the damping element (70) comprises a spring element which is preloaded when the add-on module is connected to the injection device.

7. Add-on module according to any of claims 1 to 6, **characterized in that** the first module part has an interior space and **in that** the second module part performs the damped relative movement within this interior space.

8. Add-on module according to any of claims 1 to 6, **characterized in that** the first module part comprises a receiving unit (40) and the second module part comprises a module housing (20) which surrounds the receiving unit.

9. Add-on module according to claim 8, **characterized in that** the second module part has a handle for grasping the injection device and the add-on module.

10. Add-on module according to claim 5 and according to claim 8 or 9, **characterized in that** the stop (24) of the second module part can strike a delay end stop of a needle cap remover (12) of the injection device at the end of the damped relative movement of the module parts.

11. Add-on module according to any of claims 8 to 10, **characterized in that** the the second module part comprises the release unit (50),
the add-on module (2) limiting a distal movement of the receiving unit (40) in the module housing (20) in a holding configuration by means of the release unit (50) and the holding arm (43.1, 43.2) holding the injection device (1) in the receiving unit (40);
the add-on module (2) releasing the receiving unit (40) from the release unit (50) and releasing the injection device (1) from the holding arm (43.1, 43.2) in a release configuration,
the add-on module (2) being adjustable from the holding configuration to the release configuration by means of a release movement of the receiving unit (40).

12. Add-on module (2) according to claim 11, **characterized in that** the release unit (50) comprises a guide cam (52) and the receiving unit (40) or the injection device (1) comprises a locking cam (42), the guide cam (52) assuming a holding position in the holding configuration and interacting with the locking cam (42) in such a way that the receiving unit (40) and the injection device (1) are prevented from moving in a distal direction.

13. Add-on module (2) according to claim 12, **characterized in that** the guide cam (52) can be adjusted from the holding position to a release position in which the locking cam (42) is released from the guide cam (52) and it being possible for the guide cam (52) to be preloaded into its holding position by a release button spring (80).

14. Add-on module according to any of claims 8 to 13, **characterized in that** the add-on module comprises a release support (55, 56) which is first tensioned when a or the release unit (50) is actuated and is then relaxed by shifting the receiving unit (40) relative to the module housing (20).

15. Injection device (1) having an add-on module (2) according to any of claims 1 to 14, **characterized in that** the first module part comprises two holding arms (43.1, 43.2) which are designed to engage form-fittingly in recesses in the injection device in order to establish the axially fixed connection between the injection device (1) and the first module part.

## Revendications

1. Module supplémentaire (2) électronique destiné à être monté sur un appareil d'injection (1) avant le début d'une injection, comportant un axe longitudinal reliant une extrémité côté insertion à une extrémité proximale opposée de l'appareil d'injection, comprenant un élément formant capteur (41a, 41b) destiné à détecter un état de l'appareil d'injection, un élément formant processeur (64a, 64b) destiné à analyser un signal de l'élément formant capteur, et un accumulateur d'énergie (62, 63) destiné à alimenter en énergie l'élément formant processeur, dans lequel le module supplémentaire (2) comprend en outre
- une première partie de module (40) qui comprend un mécanisme de maintien, lequel comprend un bras de maintien (43.1, 43.2), dans lequel la première partie de module (40) peut être reliée, à l'aide du bras de maintien, à l'appareil d'injection de manière fixe axialement dans la direction de l'axe longitudinal au moyen d'une saillie (44.1, 44.2) pour la mise en prise dans un évidement de l'appareil d'injection, et
- une seconde partie de module (20, 30, 50, 60),
**caractérisé en ce que** la seconde partie de module est mobile parallèlement à l'axe longitudinal dans une direction distale de l'axe longitudinal pour un mouvement relatif par rapport à la première partie de module reliée de manière fixe axialement à l'appareil d'injection, dans lequel le mécanisme de maintien reste actif indépendamment dudit mouvement relatif jusqu'à l'actionnement d'une unité de détachement (50), de sorte que la première partie de module reste reliée de manière fixe axialement à l'appareil d'injection pendant le mouvement relatif et que ledit mouvement relatif n'entraîne pas le détachement du module supplémentaire (2) de l'appareil d'injection (1), et par
- un élément d'amortissement (70) destiné à amortir le mouvement relatif.

2. Module supplémentaire selon la revendication 1, **caractérisé en ce que** l'élément d'amortissement (70) est disposé directement entre les deux parties de module.

3. Module supplémentaire selon la revendication 1, **caractérisé en ce que** la seconde partie de module présente une masse supérieure à celle de la première partie de module.

4. Module supplémentaire selon la revendication 1, **caractérisé en ce que** l'élément formant capteur (41a, 41b) est disposé dans la première partie de module.

5. Module supplémentaire selon la revendication 2, **caractérisé en ce que** la seconde partie de module présente une butée (24) qui, à la fin du mouvement relatif amorti, peut venir en butée contre une butée de fin de course de ralentissement de l'appareil d'injection ou de la première partie de module.

6. Module supplémentaire selon la revendication 2, **caractérisé en ce que** l'élément d'amortissement (70) comprend un élément élastique qui est précontraint lors de la liaison du module supplémentaire à l'appareil d'injection.

7. Module supplémentaire selon l'une des revendications 1 à 6,
**caractérisé en ce que** la première partie de module présente un espace intérieur **et en ce que** la seconde partie de module réalise le mouvement relatif amorti à l'intérieur dudit espace intérieur.

8. Module supplémentaire selon l'une des revendications 1 à 6,
**caractérisé en ce que** la première partie de module comprend une unité de réception (40) et la seconde partie de module comprend un boîtier de module (20) qui entoure l'unité de réception.

9. Module supplémentaire selon la revendication 8, **caractérisé en ce que** la seconde partie de module présente une poignée permettant de saisir l'appareil d'injection et le module supplémentaire.

10. Module supplémentaire selon la revendication 5 et selon la revendication 8 ou 9, **caractérisé en ce que** la butée (24) de la seconde partie de module peut venir en butée, à la fin du mouvement relatif amorti des parties de module, contre une butée de fin de course de ralentissement d'un dispositif d'enlèvement de capuchon de protection d'aiguille (12) de l'appareil d'injection.

11. Module supplémentaire selon l'une des revendications 8 à 10,
**caractérisé en ce que** la seconde partie de module comprend l'unité de détachement (50), dans lequel
le module supplémentaire (2), dans une configuration de maintien, limite, au moyen de l'unité de détachement (50), un mouvement distal de l'unité de réception (40) dans le boîtier de module (20), et le bras de maintien (43.1, 43.2) maintient l'appareil d'injection (1) dans l'unité de réception (40) ;
le module supplémentaire (2), dans une configuration de libération, libère l'unité de réception (40) de l'unité de détachement (50) et l'appareil d'injection (1) du bras de maintien (43.1, 43.2),
dans lequel le module supplémentaire (2) peut être déplacé de la configuration de maintien à la configuration de libération par un mouvement de libération de l'unité de réception (40).

12. Module supplémentaire (2) selon la revendication 11,
**caractérisé en ce que** l'unité de détachement (50) comprend une came de guidage (52) et l'unité de réception (40) ou l'appareil d'injection (1) comprend une came de blocage (42), dans lequel, dans la configuration de maintien, la came de guidage (52) occupe une position de maintien et coopère avec la came de blocage (42) de telle sorte que l'unité de réception (40) et l'appareil d'injection (1) ne peuvent pas effectuer un mouvement dans la direction distale.

13. Module supplémentaire (2) selon la revendication 12, **caractérisé en ce que** la came de guidage (52) peut être déplacée de la position de maintien à une position de libération, dans laquelle la came de blocage (42) est libérée de la came de guidage (52), et dans lequel la came de guidage (52) peut être précontrainte dans sa position de maintien par un ressort de bouton de détachement (80).

14. Module supplémentaire selon l'une des revendications 8 à 13,
**caractérisé en ce que** le module supplémentaire comprend un dispositif d'assistance à la libération (55, 56) qui, lors de l'actionnement d'une ou de l'unité de détachement (50), est d'abord tendu, puis se détend ensuite sous l'effet du déplacement de l'unité de réception (40) par rapport au boîtier de module (20).

15. Appareil d'injection (1) comportant un module supplémentaire (2) selon l'une des revendications 1 à 14, **caractérisé en ce que** la première partie de module comprend deux bras de maintien (43.1, 43.2) qui sont conçus de sorte qu'ils peuvent venir en prise par complémentarité de forme dans des évidements dans l'appareil d'injection afin d'établir la liaison fixe axialement entre l'appareil d'injection (1) et la première partie de module.
